# EUROPEAN PATENT SPECIFICATION

(11) **EP 3 568 700 B1**
(45) Date of publication and mention of the grant of the patent: **28.09.2022**
(21) Application number: 18738875.6
(22) Date of filing: 16.01.2018
(51) Int. Cl.: G01N 33/68, A61B 17/43

(54) **METHODS FOR IMPROVING FERTILITY IN ARTIFICIAL INSEMINATION**
VERFAHREN ZUR VERBESSERUNG DER FERTILITÄT BEI DER KÜNSTLICHEN BEFRUCHTUNG
MÉTHODES D'AMÉLIORATION DE LA FERTILITÉ DANS LE CADRE DE L'INSÉMINATION ARTIFICIELLE

(30) Priority: 15.01.2017 US 201762446455 P
(43) Date of publication of application: 20.11.2019
(73) Proprietor: Arex Life Sciences, LLC, Watertown, MA 02472 (US)
(72) Inventor: COHEN, Barb, A., Watertown, MA 02472 (US)
(74) Representative: Kasche & Partner
(86) International application number: PCT/US2018/013898
(87) International publication number: WO 2018/132838

(56) References cited:
- WO-A1-97/40386
- WO-A1-2011/048806
- WO-A1-2013/022354
- CA-A1- 2 772 889
- US-A1- 2003 215 783
- US-A1- 2012 252 000
- US-A1- 2016 033 483

## Description

### CROSS-REFERENCE TO RELATED APPLICATIONS

This application claims the benefit of priority to U.S. Provisional Patent Application Serial No. 62/446,455, titled: Methods for Improving Fertility in Artificial Insemination, filed: 15 January 2017.

### FIELD OF THE INVENTION

This application is directed to *in vitro* methods for determining an optimum time for utilizing a semen sample for insemination in assisted reproduction or storage

### BACKGROUND

Treatment of mammalian semen to achieve a higher proportion of fertility in artificial insemination can be advantageous, particularly for couples who have tried to have offspring unsuccessfully by natural means. Infertility affects 10-15% of couples in the US, according to the Department of Health and Human Services, at an incalculable cost of human suffering. According to a 2015 report of the Practice Committee of the American Society for Reproductive Medicine (Fertility and Sterility vol 103), a male factor is contributory to 30-40% of infertility in couples. In a more recent publication by Agarwal (2015), male factor infertility was responsible for 20-30% of infertility cases and contributory in 50% of cases. Compounding these difficulties are the increasingly late times in life when people choose to bear children, which creates advanced maternal age issues, as well as paternal age issues.

In addition, achieving good fertility by increasing the quality of sperm used in artificial insemination is considered to be the single greatest determinant of the success or failure of dairy farms. Because "open" or non-pregnant cows do not lactate and are therefore not productive, they decrease profit. Consequently, any increase in fertility is considered worthwhile. Similar situations exist for other types of animals raised for dairy or for meat, where offspring numbers drive profitability. Livestock includes many species such as goats, sheep, cattle, buffalo, camels, swine, etc. There is also a long-felt need in the art to improve male-side fertility in exotic or endangered species.

In US 9383369, methods of process control are described for processing semen for use in AI that particularly enable more consistent results for selection of field traits previously desired but unattainable without this process control. The methods include monitoring of the in-process collected semen in real time for processing for AI. The methods provide a solution to the impediment to desired results because of variability in the timing by which groups of sperm activate metabolically, which has hindered the development of a time-based assay for obtaining a semen sample having a desired trait, e.g., ability to fertilize an egg. It has also hindered production of sperm doses having uniform properties.

Specifically, the methods described involve monitoring, in real-time, changes in the metabolic status of sperm in a semen sample. A real-time assay of the metabolic status of sperm in an individual semen sample allows a better, more consistent determination of when, after collection of an individual sperm sample, the sperm in the semen sample have the desired traits, e.g., fertility. Such methods have found significant utility in dairy farming where prize bulls are used to inseminate herds of dairy cows. Such bulls are of proven fertility and are genetically relatively homogenous, kept in completely identical controlled housing and feeding with regular vet care making semen processing easier. Also, the assay is conducted on the exact semen that will be diluted and processed into doses (straws) that are used to perform insemination.

However, in human assisted reproductive clinics, couples have generally tried to obtain pregnancy many times before being referred for an infertility workup. Infertility is generally defined by the World Health Organization as failure to achieve a clinical pregnancy after 12 months of regular unprotected intercourse. There are biological deficiencies preventing pregnancy including, for example, low quality spermatozoa. Thus, in such clinics, pregnancy typically is achieved, according to www.babycenter.com, only about 4-5% of the time using IUI and 7-16% using IUI plus fertility techniques. The more expensive *in vitro* fertilization (IVF) procedure achieves positive results for each woman's cycle about 5-40% of the time, depending on age (21% success per cycle for women age 38-40). It would be desirable to increase the percent of positive pregnancies using the less expensive and safer IUI procedure.

The degree of variability in time course of sperm behavior from semen samples collected from an individual makes the preferred time for sperm insemination in IUI unpredictable. In addition, the way semen is processed in the human clinic significantly complicates real-time monitoring. The assay cannot be done with sperm when they are being prepared for insemination by being centrifuged, a damaging step never used with livestock. The aliquots taken before centrifugation are used for laboratory analysis, and the sperm being assayed are thus treated differently than ones used for insemination (unlike for cattle). In addition, in human clinics the sperm used for IUI are washed into synthetic medium to prevent cramping upon their placement into the uterus, exposing the sperm to even more artificial conditions and to chemicals and environments not found in the ejaculate. Often sperm count is limiting or sperm are in other ways defective, situations never seen in livestock semen doses. Therefore, in stark contrast to work with cattle, for work in the human clinic a very different sample must be used for clinical analysis while sperm for insemination are treated very differently, causing risk of divergent results. Despite this different sample of sperm cells, they must nonetheless predict an appropriate sperm state in the cells intended for dosing (insemination), which are not assayed directly.

It is understood that sperm analysis in human assisted reproductive clinics is currently practiced by evaluation of a single sample of untreated ejaculate at a single time point. This time point generally occurs before sperm have become capable of fertilizing an egg, because freshly-ejaculated mammalian sperm are infertile. Two important pieces of information vital to accurately judging sperm health and sperm in a suitable state for IUI dosing are therefore lacking: data on sperm behavior changes with time and data on sperm behavior changes that can be provoked. These changes occur, to greater or lesser extent, depending on sperm quality and sperm state.

There are many examples of how omission of this information is deleterious across species. For example, the shelf-life of sperm doses that are not frozen, which is most of them in the pork industry and in certain regions of the world for dairy cattle, is never measured. This means some bad ejaculates may be used and negatively impact conception. In the human clinic, as aforesaid, medical decisions are based on a single set of data that are not representative of the ejaculate-to-ejaculate variation, the biological changes sperm in a single ejaculate undergo with time or with provocation by specific agents *in vitro.* This single time point method of testing obscures any understanding of how sperm change with time or whether they are healthy enough to respond upon provocation with behavior changes typical of normal sperm. Noted experts in the field have decried the uselessness of current methods of sperm evaluation in the human clinic and also have expressed concern that more technically complex methods than IUI, specifically IVF and intra-cytoplasmic sperm injection (ICSI), may carry risks to babies born from IVF and ICSI (Kobayashi, 2009, Alukal, 2008). In one case, a nurse confided to Applicant that when babies entered the neonatal ICU, a routine question was whether this was an IVF baby. The nurse said this was because IVF babies were frailer and needed to be monitored more closely by medical staff.

Clearly, methods that provide tight correlation of sperm behavior with fertility (state) are needed, as are means of increasing the fertility of sperm doses. A positive impact on medical cost reduction and medical risks to the woman and her baby can be added to the incalculable benefit derived by couples finally able to have a truly wanted child.

However, collection of such ejaculate history for couples desiring an offspring is not as easily available as with prize bulls. Further, normal variations of human life - which are not found in the controlled conditions of prize bull farms -- can make each ejaculate from the same male even more different. Further, processing of semen for IUI in the human assisted reproductive clinics includes washing of the sperm to remove natural semen fluids. Thus, the processing of human sperm for IUI dosing severely impedes testing the exact sperm to be inseminated and, instead, the process may benefit from testing of sperm in an aliquot sample and natural semen medium.

Thus, there remains a need in the art to develop a time-based assay on which one can reliably depend to provide a preferred time to inseminate sperm to obtain positive pregnancy results in human assisted reproductive clinics. Ideally, the assay can be performed on site in an IUI procedure without prior male history.

### SUMMARY

The present invention is defined in the appended claims. Described are methods that enable insemination at a preferred sperm state more compatible with conception in IUI, providing increased positive pregnancy results. As such, the description provides methods of process control for processing semen into IUI doses for use in an AI clinic. The methods described herein involve monitoring, in real-time, changes in the metabolic status of successively-activating groups of sperm in a specific semen sample. The real-time assay of the metabolic status of active sperm groups in the specific semen sample allows a better, more consistent determination of when, after collection of the individual sperm sample, the sperm, or a subset (cohort, group or batch) of the sperm, in the semen sample have the desired fertility state for insemination. The real-time monitoring of the metabolic status of a group of sperm in a semen or buffer sample as they progress through their group's collective metabolic rate of sperm activation, for example, can be used to determine the real-time occurrence of expression of the fertility state in the individual semen sample, so as to provide the preferred time for insemination for positive pregnancy results. Advantageously, the methods disclosed herein can be used on site, and the increase in positive pregnancy results renders the process more economical and safer, due to lower drug exposure, than current practices. These methods allow insemination without use of invasive egg retrieval and the attendant surgical risks to the woman.

Thus, in one aspect not covered by the claimed invention, there is a method for determining an optimum time for processing a semen sample for insemination in a female by monitoring a change in the metabolic status of sperm in the semen sample during incubation and/or processing, the method comprising the steps of:
i. providing a semen sample from a male subject to be tested and incubating the sample under controlled conditions;
ii. selecting a marker that is indicative of fertility status of sperm, wherein expression of the marker changes with time, and wherein the marker is Fc receptor (FcR) or a marker whose expression correlates with FcR expression or both;
iii. determining the level of expression by sperm in the semen sample of the FcR or a marker whose expression correlates with FcR expression or both at a plurality of time points;
iv. determining the time point at which the sperm in the semen sample express the FcR or a marker whose expression correlates with FcR expression or both at about a maximum or a predetermined level that correlates to approaching a maximum of FcR expression of the sperm; and
v. selecting a time point which occurs before the sperm display a minimum level of expression of the Fc receptor (FcR) or a marker whose expression correlates with FcR expression or both, and thereby providing an optimum time for processing the semen sample for insemination to provide improved pregnancy results.

In certain embodiments not covered by the claimed invention, the method further includes the step of preparing sperm in the semen sample for insemination so that the preparation is complete and insemination can occur during a period of time near when expression of the Fc receptor (FcR) or a marker whose expression correlates with FcR expression or both is at a minimum, thereby providing a preferred time for using the semen sample for insemination.

In another embodiment not covered by the claimed invention, the description provides a method for obtaining improved pregnancy results by a real-time based assay of a semen sample from a male prior to insemination of sperm into a female of the same species to fertilize an ovum, the time-based assay method comprising the steps of:
i. providing a semen sample from a male subject to be tested and incubating the sample under controlled conditions;
ii. selecting a marker that is indicative of fertility status of sperm, wherein expression of the marker changes with time, and wherein the marker is Fc receptor (FcR) or a marker whose expression correlates with FcR expression or both;
iii. determining the level of expression by sperm in the semen sample of the FcR or a marker whose expression correlates with FcR expression or both at a plurality of time points;
iv. determining the time point at which the sperm in the semen sample express the FcR or a marker whose expression correlates with FcR expression or both at about a maximum or a predetermined level that correlates to approaching a maximum of FcR expression of the sperm;
v. selecting a time point which occurs before the sperm display a minimum level of expression of the Fc receptor (FcR) or a marker whose expression correlates with FcR expression or both, and preparing sperm in the semen sample for insemination so that the preparation is complete and insemination can occur during a period of time near when expression of the Fc receptor (FcR) or a marker whose expression correlates with FcR expression or both is at a minimum, thereby providing a preferred time for using the semen sample for insemination; and
vi. inseminating the processed sperm into a female by IUI at a time about the time point of the minimum of FcR expression of the sperm, thereby obtaining improved pregnancy results.

The assay can be conducted on an aliquot of semen or an aliquot of washed sperm in a synthetic medium which, if desirable, allows one to assay sperm in wash medium and follow groups of activating sperm in the wash medium to determine the time for insemination.

In a preferred embodiment not covered by the claimed invention, a method for impregnating a female with newly collected semen ejaculate at an optimum time for obtaining improved positive pregnancy results comprises the steps of:
i. collecting a semen ejaculate;
ii. removing a small portion or aliquot of the collected semen for assaying of sperm of said ejaculate over time for expression of a Fc receptor (FcR) marker indicative of a fertility state of said sperm;
iii. determining the level of expression by sperm in the aliquot of the FcR or a marker whose expression correlates with FcR expression or both at a plurality of time points;
iv. determining the time point at which the sperm in the semen sample express the FcR or a marker whose expression correlates with FcR expression or both at about a maximum or a predetermined level that correlates to approaching a maximum of FcR expression of the sperm; and
v. selecting a time point which occurs before the sperm display a minimum level of expression of the Fc receptor (FcR) or a marker whose expression correlates with FcR expression or both,
vi. processing remaining sperm of said ejaculate for insemination so that the preparation is complete and insemination can occur during a period of time near when expression of the Fc receptor (FcR) or a marker whose expression correlates with FcR expression or both is at a minimum, wherein after said processing, the sperm of the remaining ejaculate have the desired fertility state thereby providing a preferred time for using the semen sample for insemination; and
vii. inseminating the processed sperm into a female by IUI at a time about the time point of the minimum of FcR expression of the sperm, thereby obtaining sperm with the desired fertility state at an optimum time for insemination of a female to obtain improved positive pregnancy results.

The marker can include, but is preferably not limited to, acrosome length or morphology, appearance of the acrosomal membrane or components thereof, which appear in a punctate pattern visible through fenestrations in the plasma membrane, such pattern starting at the acrosomal rostrum and extending further down the head, expression of a cell surface molecule of sperm of the semen sample, electrostatic charge of sperm of the semen sample, and permeability of a dye by sperm or fragments thereof of sperm of the semen sample or of sperm-derived agents (such as FcR) shed into the medium surrounding the sperm, expression of a cell surface molecule of sperm of the semen sample, electrostatic charge of sperm of the semen sample, and permeability of a dye by sperm or fragments thereof of sperm of the semen sample. A preferred marker is an Fc receptor on the surface of the sperm head. As long as the marker used can be correlated to FcR expression, it can be used in accord with the present invention.

In another embodiment not covered by the claimed invention, the semen sample is assayed for said marker at intervals ranging from 1, 2, 3, 4, 5, 10, 12, 15, 20, 25 and 30 minutes, which can depend on the rate of change of expression of the marker, on the time required to perform the assay, or both. In certain preferred embodiments, the semen sample is assayed at 15 or 30 minute intervals. In a further aspect, the assay preferably utilizes an aliquot of the semen sample or an aliquot of sperm producing results correlated to the sperm in the dose.

In another embodiment not covered by the claimed invention, the sperm sample is incubated at a constant temperature during the monitoring of the marker for the metabolic status.

In another embodiment not covered by the claimed invention, the marker is selected from, though preferably not limited to, a ligand, a lectin, an enzyme, a receptor, which is expressed on the surface of the sperm, or internally or both. In one embodiment, a ligand includes, but preferably is not limited to, a protein, a glycoprotein, a carbohydrate and a glycolipid and a lipid, including ones detected by relative membrane fluidity rather than lipid molecular structure, as reflected for example in relaxation times of fluorescence polarization measurements (as reflected by the appropriate labeled lipid probe), to distinguish fluidity or different membrane regions, for example lipid rafts. In another embodiment not covered by the claimed invention the marker is selected from, though preferably not limited to, acrosome length, acrosome morphology, acrosome ruffling, expression of peanut agglutinin-positive target, expression of a cell surface molecule, electrostatic charge of said sperm, and permeability of sperm membrane, a lipid, cholesterol, phosphatidylserine, a sugar and a protein, an intracellular ion, and bicarbonate. As aforesaid, a preferred marker is Fc receptor on the sperm head surface. Any marker can be used that can be correlated to the state of change of sperm and to changes in the expression of the Fc receptor on sperm.

In one embodiment not covered by the claimed invention, binding of a first ligand or interaction of sperm with an agent evokes the appearance of a secondary marker, which is detected by contacting a secondary marker with a supplemental ligand. Alternatively, assays can be conducted with agents that do not bind, but enter the sperm or interact in some other way (such as lipid insertion into one of the lipid bilayers on the sperm or movement of agent(s) through channels, for example ion channels) to provoke a change with time that can be monitored, preferably for a detectable, preferably visible change.

In another embodiment not covered by the claimed invention, the permeability of a dye by said sperm or fragments thereof is assayed in an aliquot of said sperm sample by monitoring by the intensity of said dye in the sperm or fragments thereof. Sperm are known to bind seminal plasma agents onto the sperm membrane. Applicant has observed data that suggest that sperm can imbibe or phagocytize agents from their surrounding medium. Therefore, fragments of sperm should be construed to include agents sperm acquire as cargo, not just sperm as formed in the testis or epididymis.

In another embodiment not covered by the claimed invention, the expression of a cell surface marker is monitored, and the time point selected to process the semen sample is determined with respect to the earlier time point when expression of said marker in said sample has decreased relative to a peak expression and subsequently begins to increase from a minimum in the expression.

In one embodiment not covered by the claimed invention, the assay is based on the percentage of sperm in the semen sample having a specified marker. In one aspect, the specified marker is a biochemical marker, which is optionally present on the cell surface. Regardless, the marker reflects or is indicative of the metabolic fertility status of the sperm. The marker is not limited to a sperm specific marker.

In one embodiment, the assay encompasses determining the percentage of sperm in the semen sample having a marker that reflects the metabolic fertility status of the sperm. The assay comprises the steps of a) removing an aliquot from the semen sample; b) contacting the aliquot with a first ligand to the marker; c) detecting binding of said ligand by said sperm; and d) determining the percentage of sperm in said aliquot which binds the ligand. In an alternative aspect of this embodiment, step d) can be replaced by the step of assessing the level of the marker by a detectable label which binds the marker or the ligand either directly or indirectly.

In another embodiment not covered by the claimed invention, the assay encompasses determining the concentration of said marker detected in sperm of said semen sample comprising: a) removing an aliquot from the semen sample; b) contacting the aliquot with a first ligand to said marker; c) detecting binding of said ligand by said sperm; and d) determining the amount of marker expressed by sperm in said aliquot by quantitating the binding of the marker by the ligand; thereby determining the concentration of said marker detected in sperm of said semen sample.

In certain embodiments not covered by the claimed invention, the ligand is labeled with a detectable label, preferably a visable label. In certain embodiments, the ligand is a labeled antibody; e.g., a labeled anti-FcR antibody or a labeled antibody which binds to an anti-FcR antibody.

In another embodiment of this assay not covered by the claimed invention, detecting the binding of the ligand by the sperm includes detecting label bound directly or indirectly to the sperm, or fragments of the sperm. In one aspect, a sperm fragment is either associated or disassociated from intact sperm.

In another embodiment not covered by the claimed invention, detecting the binding of the ligand by the sperm encompasses contacting the sperm in the aliquot with a second ligand which binds to the first ligand.

In another embodiment not covered by the claimed invention, the first ligand and/or the second ligand is an antibody. The antibody can be either polyclonal or monoclonal antibodies, and can comprise one or more labels.

In another embodiment of the methods and assays described herein not covered by the claimed invention, an indicator of the metabolic fertility status of the sperm is a biochemical marker. The biochemical indicator includes, but is not limited to, one or more of the following biochemical indicators: a cell surface molecule, an enzyme and a receptor.

The time-based assay can also be used as a diagnostic assay for male infertility. It also can be used to prepare frozen or frozen-thawed doses of sperm for later use for IUI to provide sperm in the right fertility state for more successful IUI pregnancy results.

### BRIEF DESCRIPTION OF THE FIGURES

The accompanying drawings, which are incorporated into and form a part of the specification, illustrate several embodiments of the present invention and, together with the description, serve to explain the principles of the invention. The drawings are only for the purpose of illustrating an embodiment of the invention and are not to be construed as limiting the invention.
**FIG. 1A** - **1B** illustrate time-based graphs of % Fc receptor positive sperm in different samples of semen from a donor.
**FIG. 2A** - **2B** illustrate time-based graphs of % Fc receptor positive sperm in different samples of semen from another donor.
**FIG. 3A** - **3D** illustrate time-based graphs of % Fc receptor positive sperm in different samples of semen from a third donor.
**FIG. 4A** - **4B** illustrate time-based graphs of % Fc receptor positive sperm in different samples of semen from a fourth donor.
**FIG. 5** is a graph of % sperm FcR positive versus time with a dashed arrow showing accumulation of shed FcR in semen for natural insemination process.
**FIG. 6** is a graph of % sperm FcR positive versus time with a dashed arrow showing accumulation of shed FcR when sperm is washed for an IUI process.
**FIG. 7** is a graph of % sperm FcR positive versus time with a dashed arrow showing the difference in accumulation of shed FcR for natural insemination versus an IUI process.
**FIGs. 8A** - **8C** are graphs illustrating % sperm FcR positive versus time for samples of abnormal sperm.
**FIGs. 9A(1)** - **9F(2)** illustrate cytometer images and readings with corresponding plots of % sperm FcR positive versus time for an exemplary time-based assay.
**FIG. 10** is a graph of % sperm FcR positive versus time illustrating period IUI for insemination in accord with the present invention.
**FIG. 11** is a graph comparing % sperm FcR positive for washed sperm and neat semen assays.

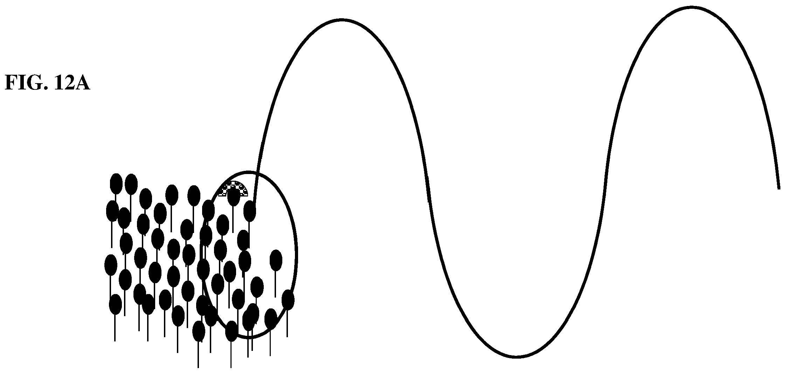

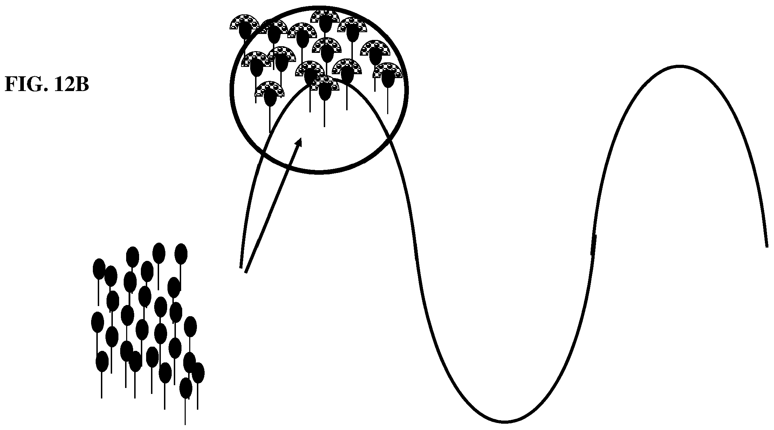

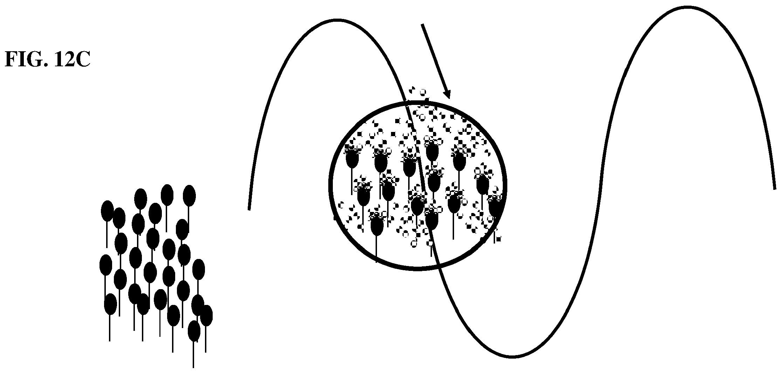

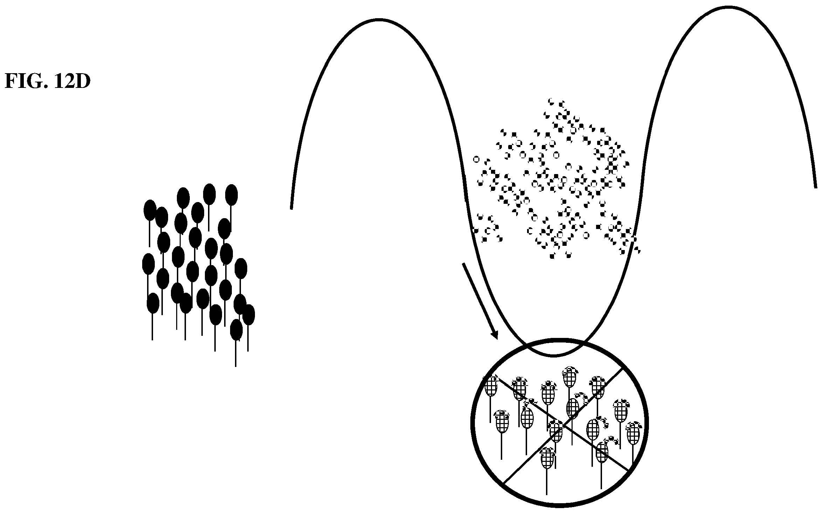

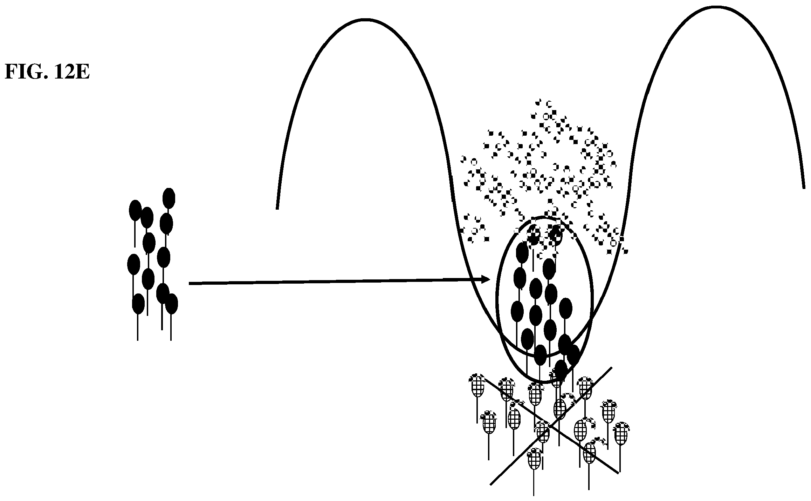

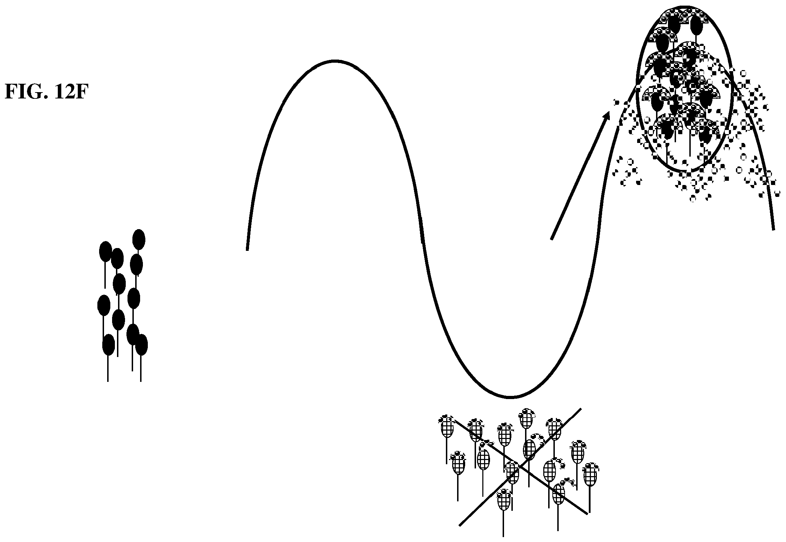

**FIG. 12A-12F** illustrates the maturation of groups of sperm in synchrony through the FcR cycle, which takes about 1h to complete for each group of sperm as they move through in succession. FIG. 12A Fresh ejaculate where all sperm are in reserve and very few are FcR positive. FIG. 12B a group of sperm in unison become positive for FcR on the surface of the acrosome. FIG. 12C the positive sperm begin shedding FcR and the sperm therefore become less positive for the FcR on their surfaces. FIG. 12D the spent sperm that have shed almost all their FcR now appear negative. FIG. 12E a new group of sperm initiates maturation from the reserve pool, in the presence of pre-existing protective shed FcR that wards off female immune attack of the foreign sperm. FIG. 12F the second cohort of sperm in unison become positive for FcR on the surface of the acrosome.

### DETAILED DESCRIPTION

The following is a detailed description provided to aid those skilled in the art in practicing the present invention.

The methods as described herein identify a fertility state window sperm normally enter in natural insemination that is interfered with by the unnatural conditions and processes to which sperm react during IUI. The methods as described herein, compensate, and enable adjustment of sperm to resemble a needed natural state for use in the clinic, preventing iatrogenic fertility failure in IUI and enabling processing of sperm doses for IUI into a uniform and preferred biological state, which is not currently possible to achieve without the instant invention.

Thus, described herein are methods that allow one to more accurately determine the time point after collection of the semen sample in which a desired fertility trait is or will be associated with the sperm relative to an earlier distinct metabolic state. The earlier distinct metabolic state need not have any relevance to the desired trait, other than it occurs at a defined number of minutes or hours before the time point during which the sperm in the semen sample have the desired fertility trait.

Unless otherwise defined, all technical and scientific terms used herein have the same meaning as commonly understood by one of ordinary skill in the art to which this invention belongs. The terminology used in the description is for describing particular embodiments only and is not intended to be limiting of the invention.

Where a range of values is provided, it is understood that each intervening value, to the tenth of the unit of the lower limit unless the context clearly dictates otherwise (such as in the case of a group containing a number of carbon atoms in which case each carbon atom number falling within the range is provided), between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

The following terms are used to describe the present invention. In instances where a term is not specifically defined herein, that term is given an art-recognized meaning by those of ordinary skill applying that term in context to its use in describing the present invention.

The articles "a" and "an" as used herein and in the appended claims are used herein to refer to one or to more than one (i.e., to at least one) of the grammatical object of the article unless the context clearly indicates otherwise. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of and "consisting essentially of shall be closed or semi-closed transitional phrases, respectively, as set forth in the United States Patent Office Manual of Patent Examining Procedures, Section 2111.03.

As used herein in the specification and in the claims, the phrase "at least one," in reference to a list of one or more elements, should be understood to mean at least one element selected from anyone or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a nonlimiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, in certain methods described herein that include more than one step or act, the order of the steps or acts of the method is not necessarily limited to the order in which the steps or acts of the method are recited unless the context indicates otherwise.

The terms "co-administration" and "co-administering" or "combination therapy" refer to both concurrent administration (administration of two or more therapeutic agents at the same time) and time varied administration (administration of one or more therapeutic agents at a time different from that of the administration of an additional therapeutic agent or agents), as long as the therapeutic agents are present in the patient to some extent, preferably at effective amounts, at the same time. In certain preferred aspects not covered by the claimed invention, one or more of the present compounds described herein, are coadministered in combination with at least one additional bioactive agent, especially including a fertility enhancing or sperm supporting agent. In particularly preferred aspects, the co-administration of compounds results in synergistic activity and/or therapy, including fertility-enhancing or sperm supporting activity.

The term "compound", as used herein, unless otherwise indicated, refers to any specific chemical compound disclosed herein and includes tautomers, regioisomers, geometric isomers, and where applicable, stereoisomers, including optical isomers (enantiomers) and other steroisomers (diastereomers) thereof, as well as pharmaceutically acceptable salts and derivatives (including prodrug forms) thereof where applicable, in context.

The term "patient" or "subject" is used throughout the specification to describe an animal, preferably a human or a domesticated animal, to whom treatment, including prophylactic treatment, with the compositions according to the present invention is provided. For treatment of those infections, conditions or disease states which are specific for a specific animal such as a human patient, the term patient refers to that specific animal, including a domesticated animal such as a dog or cat or a farm animal such as a horse, cow, sheep, etc. In general, in the present invention, the term patient refers to a human patient unless otherwise stated or implied from the context of the use of the term.

The term "effective" is used to describe an amount of a compound, composition or component which, when used within the context of its intended use, effects an intended result. The term "effective" subsumes all other effective amount or effective concentration terms, which are otherwise described or used in the present application.

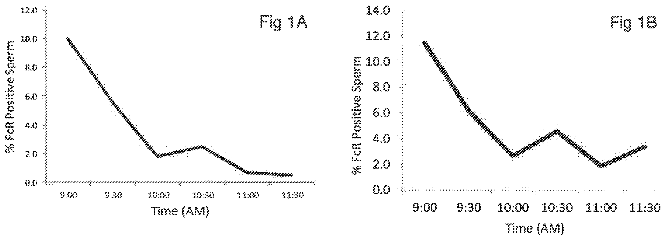

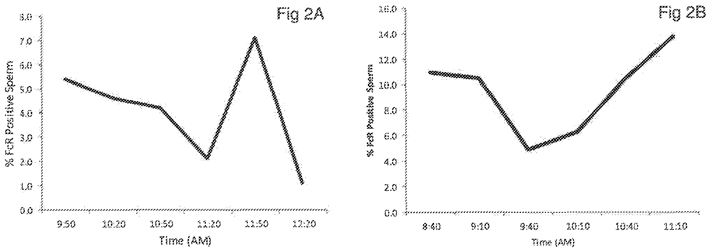

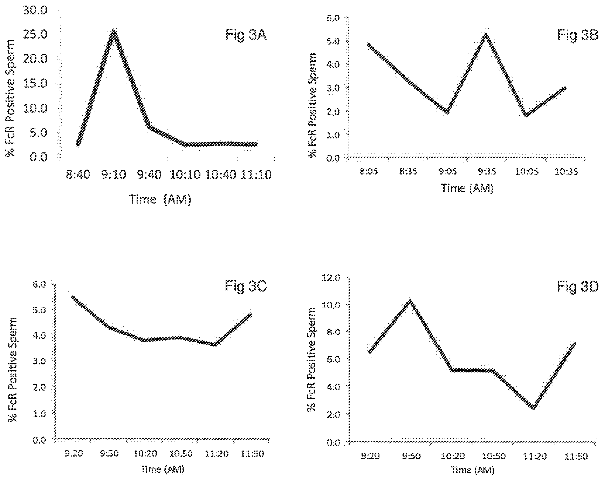

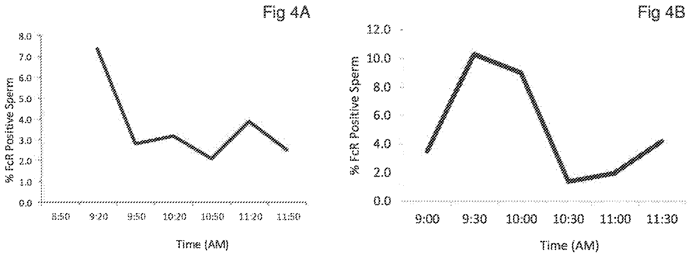

As used herein, the term "fertility trait" or "fertility state" with respect to sperm includes, but is preferably not limited to, a physiological "swim up" or motility characteristic of the sperm. The desired trait can be expressed before fertilization, e.g., the trait of being in a physiologic state capable of fertilization. The timing of the expression of the desired fertility status relative to time zero (the time of the collection) can vary significantly between semen samples, as illustrated in FIGs. 1A - 4B.

As used herein, the term "semen sample" includes any semen sample collected from an ejaculate of any mammal, including, but preferably not limited to, human, cattle, goats, sheep, buffalo, swine, horses, cats, dogs, rat, mouse, rabbits, hamsters and endangered species of mammals. Preferably the sample is incubated at controlled conditions, including incubating at a constant temperature immediately after collection and the time-based of the present invention is conducted. Semen sample includes those sample containing a portion of the elements found in a normal ejaculate, due to sample retrieval from the male tract rather than by ejaculation. This would include epididymal sperm in their surrounding fluid as obtained by needle aspiration or other procedure.

In any of the aspects or embodiments described herein, the controlled condition includes incubating the semen under a constant temperature, pH or both. In any of the aspects or embodiments described herein, the controlled conditions include incubating the semen at a temperature of from about 0° C to about 37° C. In any of the aspects or embodiments described herein, the controlled conditions include incubating the semen at a temperature of from about 20° C to about 37° C. In any of the aspects or embodiments described herein, the controlled conditions include incubating the semen at a temperature of from about 22° C to about 37° C. In any of the aspects or embodiments described herein, the controlled conditions include incubating the semen at a temperature of about 37° C.

As used herein, the term "metabolic status" or "metabolic state" or "metabolic stage" is a physiological state of the sperm with respect to specific biochemical and biophysical properties at a specific time point during the incubation period of the sperm sample. Typically, the physiology of sperm changes over time with age, such as the physiological change in fertility of sperm (see, e.g., FIGs. 1A - 4B). Thus, changes in physiology or metabolic state of sperm in a semen sample can include expression of a desired trait, e.g., fertility state. Additionally, changes in physiology or metabolic state of sperm in a semen sample can be reflected by expression of one or more markers. In one embodiment of the methods not covered by the claimed invention, the expression of one or more markers occurs earlier than the expression of the desired trait. Further, Applicant has discovered that the expression of such markers by sperm in the semen sample occurs at a predetermined time interval before the expression of the desired trait.

Thus, the phrase "a marker indicative of a metabolic fertility state of sperm" refers to a measurable attribute of a sperm, including but not limited to a physiological, structural, functional, biochemical and/or electrochemical attribute which reflects the metabolic status of the sperm at a particular time point. In accord with the present disclosure but not covered by the claimed invention, the term marker includes any attribute that correlates with the FcR expression of sperm. The term "marker" includes, but is preferably not limited to, a structural, physical, electrophysical, or biochemical attribute of sperm and/or fragments thereof, including, for example, the nonlimiting examples of acrosome length, sperm morphology (ruffling) of the sperm, expression of a cell surface molecule on the sperm, electrostatic charge of sperm, and permeability by sperm to a molecule, such as, for example, but not limited to, a dye. The term "marker" further includes, but is not limited to, a ligand, a lectin, an enzyme and a receptor, which is expressed on the surface of the sperm, or internally, or both. In some embodiments not covered by the claimed invention, the marker is a morphological change in an acrosome which can be viewed, for instance, using bright field microscopy. With respect to acrosome morphology, over time the surface of the acrosome's membrane appears increasingly ruffled. In some embodiments not covered by the claimed invention a marker can be cryptic at some stages of metabolism, and not detected.

As used herein, the term "antibody," includes, but is not limited to a polypeptide substantially encoded by an immunoglobulin gene or immunoglobulin genes, an IgG antibody, an IgM antibody, or a portion thereof, which specifically bind and recognizes an analyte, antigen or antibody. "Antibody" also includes, but is not limited to, a polypeptide substantially encoded by an immunoglobulin gene or immunoglobulin genes, which specifically binds and recognizes the antigen-specific binding region (idiotype) of antibodies produced by a host in response to exposure to the analyte. In one embodiment of the methods not covered by the claimed invention, an antibody binds the sperm or fragments thereof, or a primary antibody, through a site on the antibody other than its paratope. In another embodiment not covered by the claimed invention, the antibody binds the sperm or fragments thereof, or a primary antibody through its paratope. In another embodiment of the methods described herein, an antibody binds the sperm or fragments thereof, or a primary antibody, both through its paratope and through a site on the antibody other than its paratope.

As used herein, the term "antibody," encompasses polyclonal and monoclonal antibody preparations, as well as preparations including monoclonal antibodies, polyclonal antibodies, hybrid antibodies, altered antibodies, F(ab')₂ fragments, F(ab) fragments, Fᵥ fragments, Fc fragments (Fragment Crystallizable fragment) single domain antibodies, chimeric antibodies, humanized antibodies, dual specific antibodies, bifunctional antibodies, single chain antibodies, and the like, and functional fragments and multimers thereof, produced *in vivo* or using *in vitro* expression systems such as baculovirus, which retain specificity for an analyte or antigen. For example, an antibody can include variable regions, or fragments of variable regions, and multimers thereof, which retain specificity for an analyte or antigen. See, e.g., Paul, Fundamental Immunology, 3rd Ed., 1993, Raven Press, New York, for antibody structure and terminology. The antibody or portion thereof, may be derived from any mammalian species, e.g., from a mouse, goat, sheep, rat, human, rabbit, or cow antibody. An antibody or fragments thereof, may be produced synthetically by methods known in the art, including modification of whole antibodies or synthesis using recombinant DNA methodologies, including using phage display libraries. In preferred embodiments not covered by the claimed invention, the "antibody" binds to an Fc receptor on the surface of the sperm head or is a fragment of an antibody that possesses this binding capacity.

As used herein, the phrase "binds to" refers to an antibody, reagent or binding moiety's binding of a ligand with a binding affinity (Kₐ) sufficient to perform the assays described herein. Typically, all that is needed is weak binding, but sufficient to produce a signal. However, stronger binding affinity can be used, such that binding affinity can be at least 10³ M⁻¹, 10⁴ M⁻¹, 10⁵ M⁻¹, 10⁶ M⁻¹ or greater, preferably 10⁷ M⁻¹ or greater, more preferably 10⁸ M⁻¹ or greater, and most preferably 10⁹ M⁻¹ or greater. The binding affinity of an antibody can be readily determined by one of ordinary skill in the art (for example, by surface plasmon resonance or Scatchard analysis). A variety of immunoassay formats can be used to select antibodies specifically immunoreactive with a particular antigen. For example, solid-phase ELISA immunoassays are routinely used to select monoclonal antibodies specifically immunoreactive with an analyte. See Harlow and Lane, Antibodies: A Laboratory Manual, Cold Springs Harbor Publications, New York, (1988) for a description of immunoassay formats and conditions that can be used to determine specific immunoreactivity. Typically a binding reaction will be at least twice background signal to noise and more typically more than 10 to 100 times greater than background.

As used herein, the term "label" includes a detectable indicator, including but not limited to labels which are soluble or particulate, metallic, organic, or inorganic, and may include radiolabels (such as, e.g., ¹⁴C, ³H, ³²P), enzymatic labels (e.g., horseradish peroxidase, galactosidase, and other enzyme conjugates), spectral labels such as green fluorescent protein, fluorescent dyes (e.g., fluorescein and its derivatives, e.g., fluorescein isothiocyanate (FITC), Alexa Fluor^{®} 488 Dye, which is a green-fluorescent dyes conjugate with nearly identical spectral properties and quantum yield as fluorescein isothiocyanate, rhodamine, Yo-Pro, a carbocyanine nucleic acid stain sold by Invitrogen, catalog Product V13243, the green-fluorescent YO-PRO^{®}-1), chemiluminescent compounds (e.g., luciferin and luminol), spectral colorimetric labels such as colloidal gold, or carbon particles, or colored glass or plastic (e.g. polystyrene, polypropylene, latex, etc.) beads, as well as dyes, including the cell-permeant pH indicator, carboxy SNARF^{®}-1, an acetoxymethyl ester, acetate which has a pKa of ~7.5 after deesterification and is sold by Invitrogen, as catalog # PPLM63- C1270. Where necessary or desirable, particle labels can be colored, e.g., by applying dye to particles. In preferred embodiments the "label" is a visible label.

As used herein, the term "colored particle label" includes, but is not limited to colored latex (polystyrene) particles, metallic (e.g. gold) sols, non-metallic elemental (e.g. Selenium, carbon) sols and dye sols. In one embodiment not covered by the claimed invention, a colored particle label is a colored particle that further comprises a member of a conjugate pair. Examples of colored particles that may be used include, but are not limited to, organic polymer latex particles, such as polystyrene latex beads, colloidal gold particles, colloidal sulphur particles, colloidal selenium particles, colloidal barium sulfate particles, colloidal iron sulfate particles, metal iodate particles, silver halide particles, silica particles, colloidal metal (hydrous) oxide particles, colloidal metal sulfide particles, carbon black particles, colloidal lead selenide particles, colloidal cadmium selenide particles, colloidal metal phosphate particles, colloidal metal ferrite particles, any of the above-mentioned colloidal particles coated with organic or inorganic layers, protein or peptide molecules, or liposomes. For example, Quantum dots sold by Invitrogen, is a label encompassed herein but not covered by the claimed invention.

As used herein, the term "decreased expression" with respect to a marker, refers to a decrease in expression (including a decrease in accessibility or an increase in crypticity) of a marker or given measurable activity (e.g., binding activity, membrane permeability, electrostatic charge) by at least 5% relative to a reference. Such decreased expression or activity is down regulated by at least 5%, 10%, 15%, 20%, 30%, 40%, 50%, 60%, 70%, 80%, 90%, or more, up to and including 100%, i.e., complete absence of the given expression or activity. Decreased expression of a marker can be measured as described in the working examples herein. The term "increased expression" refers to an increase in expression of a marker or given measurable activity (e.g., binding activity, membrane permeability, electrostatic charge) by at least 5% relative to a reference, for example, at least 10%, 15%, 20%, 30%, 40%, 50%, 60%, 70%, 80%, 90%, or even 100%. An increased expression or activity of a marker can be measured as described in the working examples herein.

As used herein, the term "fertility" with respect to sperm in a semen sample, refers to the ability of the sperm at some point in its activation life cycle to fertilize an egg and create a viable fetus and/or live-born animal. This ability changes with conditions and as the sperm age.

As used herein, the term "punctate staining" means a distribution of detectable label in the form of distinct spots or points as opposed to a uniform staining across the surface of a sperm or fragments thereof. Quantitative analysis of punctate staining is known in the art, as described for example by Maxim Mokin and Joyce Keifer in "Quantitative analysis of immunofluorescent punctate staining of synaptically localized proteins using confocal microscopy and stereology", Journal of Neuroscience Methods, Volume 157, Issue 2, 30 October 2006, Pages 218-224. As a group of sperm progress through their activation cycle, labeling becomes more contiguous, resembling snow on the peaks of a mountain range as the acrosome ruffles or wrinkles.

As used herein, the term "pregnancy" refers primarily to clinical pregnancy results, meaning detection of a fetal heartbeat at 6 weeks. The term also refers to biological pregnancy, meaning an increase in the amount of human chorionic gonadotrophin (hCG) present in blood as a pregnancy marker. In home pregnancy tests, urine is the sample employed for hCG detection. In both forms of pregnancy, a sperm has successfully fertilized an egg. The sperm selected by methods of the invention are likely to be healthier and produce better embryos, which encourages maintenance of pregnancy versus spontaneous abortion.

As used herein, Fc receptor (FcR) includes, can mean unless the context clearly indicates otherwise, a biomolecule shed or secreted by sperm that binds the Fc portion of an antibody. In any of the aspects or embodiments described herein, the FcR comprises or is a lectin protein. In any of the aspects or embodiments, the FcR comprises or is a fucose-binding lectin protein.

Increased positive pregnancy results are a desired goal for assisted reproductive clinics and economically desired by couples seeking reproductive aid. It is understood that positive pregnancy results in IUI procedures typically are about 4 - 16% of the procedures, meaning couples wanting offspring typically conceive within 7 IUI cycles according to The Sperm Bank of California, each at a financial as well as emotional cost. Then, if IUI is unsuccessful, the couple must consider IVF procedures at considerably more cost, which in many cases is not covered by insurance. Improved positive pregnancy results can be obtained using the methods as described herein, which are designed to inseminate the female at a suitable sperm maturation/fertility state, thereby providing the improved pregnancy results. The described methods are based on the surprising and unexpected discovery and application of the oscillatory nature of sperm FcR cycling. The assay can be performed on a sample of sperm not used for IUI, while the sperm being used in IUI are being washed (making them inaccessible to assay) for use in insemination, or washed sperm can be assayed in the resuspension medium.

Sperm are very unusual cells in that they face a hostile environment that they must effectively overcome in order to achieve fertilization. Although not being bound by theory, it is believed that the hostile environment is deliberate in nature, as it enables selection of the best sperm into the pool that will fertilize the egg. This may explain why sperm of higher quality (having better morphology) are found in the oviduct as compared to the ejaculate (Ahlgren, 1975).

As described herein, it is hypothesized that a sperm group or cohort travels cooperatively or in synchrony through specific fertility states during maturation and life cycle. This is believed to occur through cell behavior changes that a small group of sperm, out of the entire ejaculate, undergoes in synchrony. Each successive group of sperm to undergo this is drawn from the starting pool, which accordingly shrinks as the pool of spent sperm increases in size.

Not all states are present in the ejaculate at all times. This cycling of successive sperm groups enables migration of a subset of sperm into the oviductal storage reservoirs that are a safe haven for the potential egg-fertilizing sperm. The entire process is believed to be dependent upon two parameters that are not traditionally measured when evaluating sperm fertility: (1) onset of the process by which sperm gain fertility, which occurs with variable timing post-ejaculation (freshly ejaculated mammalian sperm are infertile), and (2) improvement in fertility status of sperm in response to exogenous agents (this can be used as an index of sperm health). The ability to assay sperm for these parameters has historically been difficult or impossible. Such assay should measure the gain by sperm of attributes required for fertility to produce successful pregnancy.

Significant differences exist between a natural insemination process and a human assisted IUI clinical process. In the natural process, the male ejaculates the semen directly into the vagina. In the IUI process, washed sperm in a synthetic medium is injected into the uterus. In the natural process, semen and mucus act as protective agents as the sperm matures during deposit into the vagina and swim up into the uterus. In the IUI process, the maturation status of sperm is not known and the coordination of sperm maturation with female tract location is interfered with by washing procedures so that the sperm is introduced into the uterus at various fertility states including states incompatible with pregnancy. In the natural process semen is inseminated into the vagina and temperature of the sperm is controlled relatively at body temperature (about 37° C), whereas in the IUI process there can be multiple decreases and increases in the temperature at which sperm is kept prior to injection into the uterus. In the normal process, sperm are never exposed to light, whereas the IUI process exposes sperm to light, including UV light, which is known to damage sperm (Torres, E.R.; Abad, C., Pinero, S., Proverbio, T., Marin, R., Proverbio, F., and Camejo, M.I. (2010). Effect of ultraviolet C irradiation on human sperm motility and lipid peroxidation. Int. J. Radiat. Biol. 86(3): 187-193). In the normal process, the sperm are exposed to reduced oxygen tension due to lower amounts of oxygen in female tract and presence of semen (Fischer, 1993), whereas the IUI process exposes sperm to ambient oxygen for relatively long periods prior to injection into the uterus, potentially raising the known risk to sperm of oxidative damage (Guthrie, 2012). In some cases of IUI, only part of the ejaculate - and therefore a reduced sperm number relative to natural insemination - is used due to volume restrictions, whereas in natural insemination the entire ejaculate is deposited. In the natural process Fc receptors on the sperm are shed and are present in the female reproductive tract, whereas in the IUI process Fc receptors in the semen are washed away with semen during processing for insemination into the uterus.

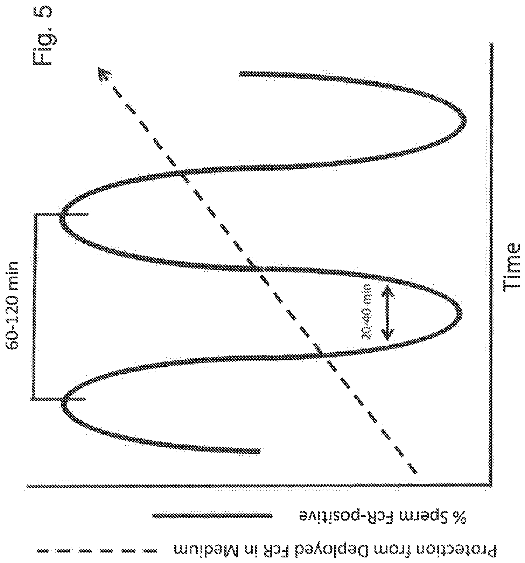

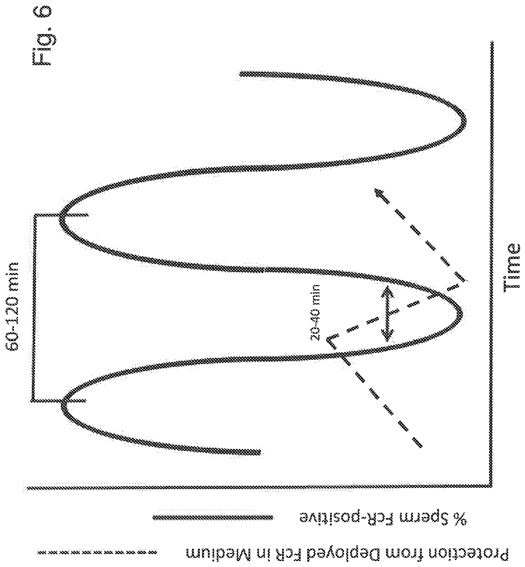

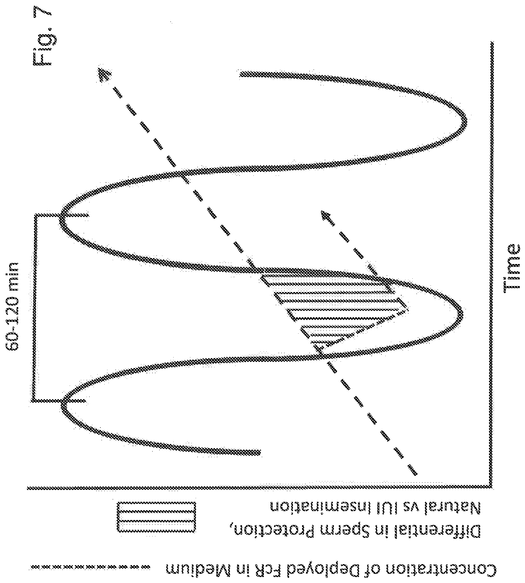

In a natural insemination process, as sperm mature, FcR are shed from the sperm head and are believed to be in the semen or in whatever environment(s) sperm find themselves experiencing in the female tract fluids when FcR are deployed. See FIG. 5 where the dashed arrow illustrates the increase of FcR in semen as cohorts of sperm mature while the curve illustrates % FcR positive sperm vs. time. However, in the IUI process, the washed sperm eliminates FcR in the semen and solely FcR shed after washing begin to accumulate in the synthetic medium (see FIG. 6, where the dashed arrow illustrates the level of FcR in the medium over time). FIG. 7 illustrates a significant difference that can occur between FcR shed in semen inseminated in the natural process versus shed FcR in the medium inseminated in the IUI process. This difference can be expected to occur to various extents, based on the relationship between washing and sperm state. If sperm deploy before being resuspended in the insemination medium, FcR will be low until the next cohort of sperm initiates (Figure 7). If sperm deploy in the insemination medium, then FcR from a single group (but not all of the groups) will be present. This contrasts to the normally larger amount of FcR present from multiple groups of sequentially activating sperm that is found in natural insemination.

In a natural insemination process, as sperm mature, FcR are shed from the sperm head and are believed to be in the semen or in the female tract fluids. For example, Figure 5 shows the increase in of FcR in semen as cohorts of sperm mature (dashed arrow), while the curve illustrates % FcR positive sperm versus time. However, in the IUI process, the washed sperm eliminates FcR in the semen and solely FcR shed after washing begin to accumulate in the synthetic medium (see FIG. 6, where the dashed arrow illustrates the level of FcR in the medium over time). FIG. 7 illustrates a significant difference that can occur between FcR shed in semen inseminated in the natural process versus shed FcR in the medium inseminated in the IUI process. This difference can be expected to occur to various extents, based on the relationship between washing and sperm state. If sperm deploy before being resuspended in the insemination medium, FcR will be low until the next cohort of sperm initiates (Figure 7). If sperm deploy in the insemination medium, then FcR from a single group (but not all of the groups) will be present. This contrasts to the normally larger amount of FcR present from multiple groups of sequentially activating sperm that is found in natural insemination.

At the time of collection, the quality and maturation of sperm can vary considerably. Generally, it is considered that in many cases, healthy sperm are dormant and groups of sperm sequentially undergo metabolic changes enabling the groups of sperm to become activated one after another over time. After reaching maximum activation, the sperm continue with their aging process, becoming less fertile, and eventually degrading. However, as a result of research in the field, it is hypothesized that a healthy semen sample has multiple groups (sub-populations) of sperm capable of activation and that the groups of sperm activate during different periods of time and that activation and aging of one group typically is followed by activation and aging of a second group of sperm, often followed by a third group, and possibly more groups (see Figure 12A-12F). In the methods described herein, the time based assay detects groups of sperm sequentially by cycles of FcR (or marker) deployment to provide a meaningful improvement in IUI reproductive outcome.

The time course of activation of sperm from its dormant state at collection varies not only among individuals within the same species, it also varies between ejaculates obtained from the same individual. See FIGs. 1A - 4B. Conventional wisdom in the art was that this large degree of variability in the time course of sperm activation made it difficult or impossible to reliably assess the processes of sperm or to obtain a semen sample with physiology characteristic of a certain time in development such as its fertility based solely on the time interval after collection of the semen sample.

In conventional semen processing, semen is collected and treated in one of two ways for intrauterine insemination (IUI). For immediate use, seminal plasma is washed from sperm and the sperm are prepared for IUI within hours of collection. For later use, semen is collected, diluted in an artificial medium and processed into frozen doses. A difficulty with these procedures is that they risk introduction of iatrogenic IUI infertility by not coordinating insemination with the changing fertility state of sperm in the ejaculate. Sperm must be inseminated during IUI at a specific biological fertility state (previously not measurable) or pregnancy will not result. Disclosed but not claimed is a method to measure the changing sperm state, post-collection. This enables IUI to occur at the sperm state permissive for pregnancy and enables avoidance of states that will fail to produce pregnancy.

In processing in accord with the methods of the present description not covered by the claimed invention, semen is collected and processed in accord with clinical procedures (with sample production preferably at the clinic, not using a sample produced at home and transported), with the exception that an aliquot (e.g., about 50 µl) that will not be used for IUI is removed for assay. The assay data can be interpreted two ways. It can be used diagnostically to identify men whose sperm are unable to produce the normal sperm state change required for IUI pregnancy. The data can also be used for manufacturing process control to make sperm doses that will be at the correct sperm fertility state to be more likely to produce pregnancy upon IUI. This includes fertility state adjustment to an IUI-compatible state of: (1) sperm used for liquid semen doses, (2) sperm state in thawed semen doses prior to IUI with a dose that was initially in the frozen state and (3) preparation of sperm doses for freezing to await later thawing and IUI.

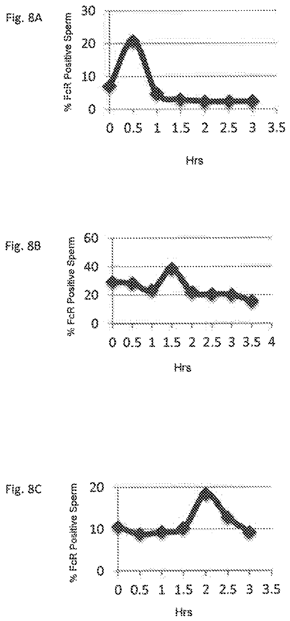

Figs. 8A - 8C illustrate graphs for time-based assays showing sperm of donors where the sperm exhibit characteristics that make the sperm highly unlikely to produce pregnancy. Because ejaculate quality can vary between ejaculates from the same male, this understanding of ejaculate quality can be used to improve chances of IUI pregnancy achievement, for example, by performing two IUIs with ejaculates produced on sequential days if the first day ejaculate is deemed questionable. Thus, use of the time-based assay can diagnose sperm capability of quality for producing pregnancy in IUI treatments that demand more of sperm performance, in contrast to risky techniques that require surgery, such as IVF and ICSI. For IVF and ICSI, use of time-based assay can still diagnose sperm fertility state, however, different diagnostic criteria would be necessary to provide suitable state. Significantly, the described methods allow identification of single cohort ejaculates, where only a single group of sperm mature (these are the subject of Figures 8A - 8C), and as described herein, the same male can produce single and multi-cohort (Figures 1A - 4B). So, the described methods can improve the chances of fertilization by eliminating low-probability-of-pregnancy ejaculates and providing the option of multiple IUIs per cycle to improve ejaculate quality per cycle if this is indicated (i.e., by production of a single cohort ejaculate for IUI).

Assays in accord with the present invention allow compensation for the changes introduced by IUI that differ from natural insemination. Without sperm fertility state adjustment, these changes might otherwise lead to introduction of sperm, into the highly selective uterine environment, at a state that is incompatible with production of pregnancy.

With sperm fertility state adjustment, it may be possible with sufficient cell number and normalcy to dilute an ejaculate and perform IUI directly, as sperm will be in the correct state to reach the oviductal storage reservoirs. However, the acceptable sperm fertility state window is narrow and, generally, it is not possible to detect the correct state, then to process semen and to complete IUI in such a narrow time window. Hence, the predictive value of assays in accord with the present invention in anticipating the sperm fertility window and allowing adequate preparation time is very useful. In published material that speaks to the number of sperm required to produce pregnancy, IUI-compatible state has never been recognized as a confounding variable. For this reason, Applicant believes a number of couples previously treated by IVF due to IUI cycle failures (many being iatrogenic, or medically-caused) or due to oligozoospermy (low sperm count) now can successfully achieve pregnancy through use of the instant invention.

In certain embodiments not covered by the claimed invention, the ejaculate can be separated into aliquots and assayed. As each aliquot reaches approximately a suitable compatible fertility state (a preferred sperm fertility state revealed by the assay to be suitable for production of pregnancy) at different times, these aliquots can be sequentially inseminated at the correct time, for a further enrichment of sperm number in the face of oligozoospermy. This process enables IUI with successive groups of activating sperm, because they are known to be activating by in vitro assay with the instant invention. It is hypothesized that different suitable compatible fertility states ("optima") may exist for different fertilization modalities, which are expressly contemplated herein. Therefore, unless the context indicates otherwise, the term "preferred" or "preferred metabolic state" or "preferred compatibility" as used herein refers to the suitable compatible fertility state for pregnancy by a particular fertilization method.

Sperm undergo a maturation process upon ejaculation. As described herein, sperm mature in successive groups (cohorts) as determined by FcR biochemistry. This is consistent with fully mature activated sperm having a short life during which they can fertilize an egg, or they will die (Cohen-Dayag, 1995). Therefore, assays in accord with the present description detect, or correlate with, the presence of the sperm FcR expression which appears on the sperm head during sperm maturation and then is shed from sperm, with timing of maturation and fertility state specific to each ejaculate.

For example, an initial cohort of sperm becomes positive then sheds the protective FcR marker. Then, typically at intervals of 1-2 hour(s) another group of sperm matures. Thus, the FcR marker can be used to determine the maturation, and thus fertility, status of a group of sperm being used therapeutically. The marker can also determine the capacity of sperm in an ejaculate to undergo maturation with correct timing, in a nonlimiting example, with respect to normal number of groups, normal shape of assay curve, normal kinetics of FcR initial expression and percent of population expressing, in a diagnostic setting. With this knowledge, it is possible now to time sperm dose processing, to biologically adjust a semen sample for fertility state, or to stratify patients into pre-ART (IUI) or ART (Assisted Reproductive Technologies) procedures based on sperm fertility state.

Even though maturation of sperm groups can vary significantly from individual to individual and even for different ejaculates from the same individual, it was surprisingly discovered that the time interval between an earlier metabolic state reflected by maximum expression of the Fc receptor (FcR) marker and the later desired fertility state trait is relatively consistent for semen samples collected from individuals and incubated under the same conditions. This consistent time interval between movement through fertility states by a group of sperm is used in the real-time methods described herein to determine when sperm of each individual semen sample will express the desired fertility state by monitoring expression of one or more markers. The time at which the semen sample has the desired fertility state and can be processed for artificial insemination, for example, IUI, can be determined by monitoring marker expression and processing at the time at which the sperm in the semen sample express the marker at a specific level.

Metabolic state predicts the fertility state. The concept extends biomarker detection to process control, specifically, for a group of cells that move from dormancy to activity then to senescence in ways that affect their field performance and can now be measured in real time prior to sperm use. Preferably, the defined metabolic state is easily identified by monitoring for the co-expression of a specified marker(s), or for a specific level of expression of a specified marker(s). Once it is determined the time at which a group of sperm in the semen sample achieved the desired metabolic state, one can determine the later time point when sperm in the semen sample will have the desired fertility state, by applying the predetermined defined time interval between expression of the label and the desired fertility trait. Thus, knowing when a group of sperm in the semen sample achieve the desired metabolic state as indicated by monitoring the expression of the marker(s), enables semen to be processed for artificial insemination (AI) that will result in higher pregnancy achievement.

Attainment of a particular metabolic state requires passage of time -- an amount of time that differs for every ejaculate, being especially variable for the first maturing group of sperm. However, Applicant has found that the time for attainment of a specific fertility state can be correlated to the expression of Fc receptor on the sperm head which is related to sperm maturity and, after maturation begins, maturation of a sperm group progresses with timing that is relatively consistent across ejaculates. Therefore, according to Applicant's working model, by evaluating the timing of metabolic states by assay, and correlating this timing to sperm behavior, it is possible to predict and obtain desired fertility states. This is achieved by using the time-based assay to dictate the timing of sperm processing, that is, when sperm are prepared for artificial insemination.

In accord with the present description, as aliquots of the collected ejaculate are assayed at preselected time intervals after collection of the semen, the expression of the Fc receptor marker by sperm fluctuates through a maximum followed by a minimum, and then increases in expression, and can cycle through maximum and minimum. The biological expression of the Fc receptor marker correlates with fertility state of the sperm. By processing the sperm at about the maximum in expression of the Fc receptor marker, the sperm can be ready for insemination at the time of near optimum fertility state occurring for IUI in a time window beginning at a time when FcR expression approaches a minimum to a time when FcR expression is increasing after going through the minimum.

In certain embodiments not covered by the claimed invention, methods described herein generally comprise collecting a semen sample. This is preferably produced at the clinic or physician's office, not produced at home and transported. In certain aspects, the semen is visually inspected for viability. The sample can be neat semen or washed sperm resuspended in supportive medium. In a preferred embodiment, reagents are stored during assay time at 22-26° C and semen or washed sperm are stored at from about ambient (e.g., room temperature or about 20° C) to about 37° C. In certain embodiments not covered by the claimed invention, the semen or washed sperm is stored at 37° C. Allow the semen to liquefy, this requires about 30 minutes from production (consistent with current procedures), but it is preferred to begin testing immediately. In certain embodiments, 50 µL sample aliquots are removed for testing. The remainder is for the insemination. In certain exemplary embodiments not covered by the claimed invention, the assay is prepared as follows:
i. place into 1.5 mL tube, 100 µL of GREEN 1, 20 µL of RED 2, mix. Add 10 µL neat semen (or washed semen), and add 5 µL BLUE 3, mix;
ii. repeat this procedure at regular intervals of every 15 or 30 minutes and reagents at or near ambient, not ice cold for assays because of the concern that cold reagents (e.g., ice) can cause an artifact with fresh semen through temperature shock, but cold reagents are better tolerated after first time point or two.

Thus, for example, in step a, one hundred (100) µl antibody diluent with Bovine Serum Albumin (Invitrogen SKU # 00-3118) (GREEN 1) is mixed with twenty (20) µl of primary antibody (rabbit anti-Salmonellas spp; Salmonella H antiserum A-Z product number 224061; BD/Difco, Detroit, MI; reconstituted according to the manufacturer's instructions or reconstituted with the phosphate buffered saline described herein made from PBT tablets) (RED 2), followed by 5 µl of secondary antibody conjugated to Alexa Fluor^{®} 488 (goat anti-rabbit IgG (H + L); Invitrogen/Thermofisher, Carlsbad, CA, catalogue no. A11008 ) (2 mg/1) (BLUE 3). Examples of other diluents (GREEN 1) useful in the practice of this invention include:
phosphate buffered saline (e.g., PBS Tablets without calcium without magnesium, MP Biomedicals LLC, cat no. 2810305 prepared according to manufacturer's instructions), HTF HEPES culture medium with EDTA and glutamine (InVitroCare catalog # 2002), Quinns (TM) Sperm Washing Medium (Origio catalog # ART-1006) and other supportive buffered cell-supportive media. Other antibodies (RED 2) include: any having an Fc fragment, or containing N-glycosylation associated with the Fc fragment of antibodies, that can be used in the assay, including: Difco antiserum for microbiological testing, for example, BD Difco Salmonella O Antiserum Poly A-I & Vi (BD Difco catalog # 222641, and ChromePure Rabbit IgG, Fc Fragment (Jackson ImmunoResearch catalog # 011-000-008), as well as IgM molecules. Other conjugants (BLUE 3) include: Goat Anti-Rabbit IgG (H + L), DyLight(TM) (ThermoScientific catalog # 35552), and secondary antibodies conjugated with FITC or other fluorophores for which cytometer excitation and emission filters are chosen to be suitable.

For each aliquot, mix (can use Vortex) for about 2 seconds and incubate for about 20 minutes. After about 20 minutes, wash with buffer and centrifuge for 30 seconds. Remove as much of the supernatant as possible without disturbing the pellet. Add more buffer, usually 100-300 ul, resuspend pellet and vortex for about 5 seconds. Place on cytometer SIP tube. Run the sample on the cytometer on an appropriate speed based on cell count, this will record data points on a BD Accuri C6 cytometer file from which a score can be interpreted.

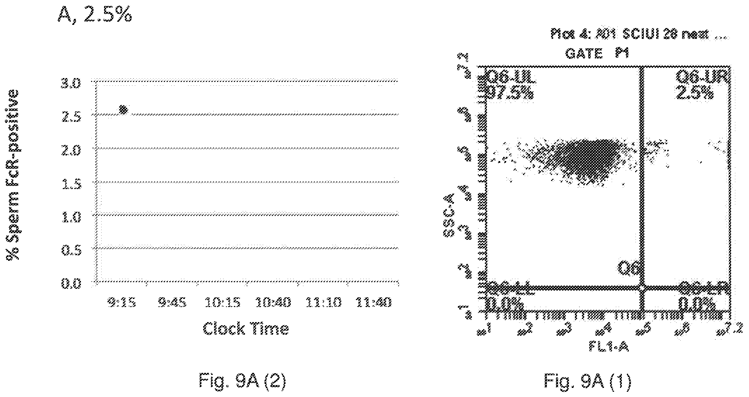

Figures 9A(1) - 9F(2) show data from a cytometer for one assay. The figures on the right show cytometer images and corresponding figures on the left show the graph of % sperm Fc receptor positive versus time as obtained from the cytometer readings.

It was surprisingly discovered that there is a relationship between expression of markers on sperm and the range (window) of preferred times for insemination of the sperm at or near the preferred fertility state. In accord with the present description, the window for insemination is a time period around a minimum in the FcR assay following a maximum, wherein the window includes about 20 minutes on either side of the minimum, preferably about 15 minutes on either side of the minimum and, more preferably about 10 minutes on either side of the minimum. The described methods allow for determination of the preferred fertility state for different ART platforms as well. Using the information about this relationship, by monitoring the expression of an Fc receptor on the sperm head, an appropriate time can be determined to perform the insemination procedure to introduce sperm at or near the preferred fertility state of the sperm group's activation or maturation cycle. This FcR expression relationship is relatively constant between individual samples from the same species incubated or held under the same conditions.

Using this relationship, real time monitoring provides a means to tailor semen processing for each individual ejaculate, so that the sperm is ready for insemination at a preferred fertility state to provide increased possibility for positive pregnancy results.

### Obtaining sperm having suitable fertility state

In accord with the present description not covered by the claimed invention, the assay involves time-based monitoring of one or more markers related to fertility state to determine when to process the semen so that the processed semen will be at or near the preferred fertility for insemination. Preferably the marker is a Fc receptor or another biological marker that can be correlated to the expression of the Fc receptor.

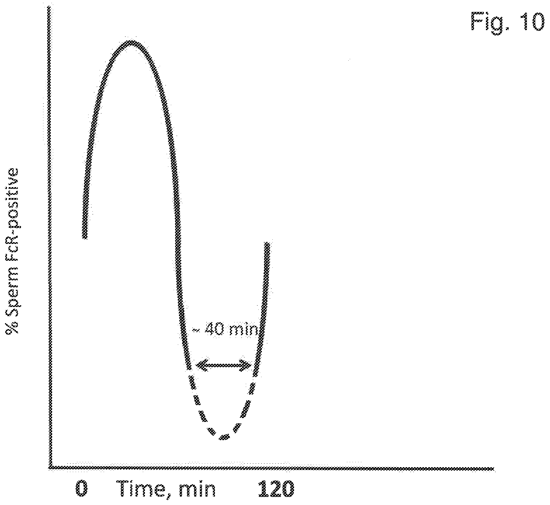

Specifically, the suitable time point for processing the sperm depends on the relationship of the time-based expression of the marker being monitored and the fertility state of the sperm. In IUI studies to date, 100% of pregnancies occurred when sperm was inseminated within a 40 minute period (window) around a minimum in % sperm Fc receptor positive in the time-based assay (see FIG. 10). Notably, monitoring swim up motility does not permit time for processing and then insemination at the proper fertility stage as determined according to the described methods. Thus, in one embodiment not covered by the claimed invention, aliquots of the sample can be removed from the semen sample of interest at various time points and assayed for expression of the specific selected marker(s) so that the time for processing can be determined. The time point for processing the sperm in the particular semen sample is then determined by time-based expression of the marker.

The monitoring of the obtained semen sample encompasses the following steps of:
i. determining the percentage of sperm in the semen sample having a marker/indicator of its metabolic status during incubation post collection at various time points, thereby providing a time-based graph of the marker expression for that semen sample; and
ii. selecting the time point at which sperm should be stabilized, such as through freezing, or utilized, to obtain semen at or near a preferred fertility state when processing is completed.

As discussed above, the time at which the sperm attains a preferred fertility metabolic status (i.e., approximately most compatible fertility state) varies from sample to sample, but can be predicted by assaying for a marker which relates to the fertility metabolic status. Aliquots can be taken from the incubated semen sample at regular intervals beginning at the time at which the semen sample is first collected. In one embodiment not covered by the claimed invention, aliquots are assayed every 5 minutes, every 10 minutes, every 15 minutes, every 20 minutes or every 30 minutes. It is preferable to take and assay aliquots at the smallest reasonable time intervals, depending on the time it takes to conduct the assay. The smaller the time intervals between assaying of aliquots, the better the ability to process the semen sample for suitable sperm fertility state, when the process is completed, for insemination to provide enhanced pregnancy results.

In fertility clinics, it can be preferred to perform the assays on aliquots of the semen sample taken soon after collection, while performing washing and other processing steps on the at least a portion of the remaining sample to be ready for insemination when the assay indicates a near minimum of FcR or other marker activity.

Monitoring of semen samples can be performed by any means known to those skilled in the art. A preferred method for monitoring is by use of a cytometer. (See FIGs. 9A(1) - 9F(2)).

### Advantages

The full advantage of increased fertility for AI procedures has been unreachable due to the lack of an effective fertility technique suitable for in-clinic on-site use. What has been needed is a method that reveals the preferred timing for insemination for improved fertility and pregnancy when used with standard clinical methods of artificial insemination. An ability to monitor sperm biological processes as they occur following semen collection for processing provides a way to tailor semen processing, including the insemination procedure, to each individual collection of semen, thereby improving sperm quality and maximizing fertility. The present assay provides a solution which makes it possible to increase fertility in methods that are applicable on-site.

### Markers of Metabolic Status

Sperm fertility state can be defined on the basis of numerous attributes such as number of viable sperm, sperm motility (both the percentage that are motile and the type of motility exhibited), sperm morphology, acrosomal integrity, etc. It is known that all sperm go through a series of metabolic changes once ejaculation has occurred and the sperm is mixed with plasma from the seminal vesicles and with other fluids. The sperm "maturation," which includes "capacitation" that follows ejaculation, is necessary for sperm to achieve fertilizing ability. A number of membrane changes are associated with these processes (Bearer and Friend (1990) J Electron Micros Tech. 16: 281-297). However, Applicant has found that the Fc receptor provides a preferred marker for fertility stage. Thus, any biological marker that can be correlated to the Fc receptor is useful in the practice of the present invention but not covered by the claimed invention.

It has been found that a preferred expression marker of fertility state is an Fc receptor (FcR) on the head of sperm. The quantity of Fc receptors expressed on the head of sperm, and/ or the number of sperm in the population expressing them, can be monitored over time to provide a plot of Fc receptor expression versus time. The expression of Fc receptors can be monitored by use of a labeled ligand that binds to the Fc receptor. Useful such ligands include labeled antibodies having no other affinity for proteins expressed or shed by the sperm or present in the sperm medium. The plot of Fc receptor expression versus time typically shows a population (group, batch or cohort) of sperm expressing Fc receptors reaching about a maximum and declining to about a minimum followed by a second population (cohort) of sperm expressing Fc to about a maximum followed by declining to about a minimum and, then, possibly even a third population (cohort) of sperm expresses FcR to about a maximum followed by declining, and possibly more cohorts.

In accord with the present description not covered by the claimed invention, when semen is collected for IUI, an aliquot is taken for time-based assaying at periodic intervals and monitoring the assay profile for fertility status. The remaining semen is processed for IUI according to standard clinical methods. It is inseminated in accord with fertility status as determined by the time-based assay and monitoring. In accord with the present invention, insemination should occur during a period of time when FcR expression is near a minimum, i.e, preferably, during a window of time starting just before a minimum FcR expression and ending just after going through a minimum. In order to be ready to inseminate at the time when the FcR expression is going through a minimum, preferably processing the remaining semen, or portion thereof, to be used for insemination should begin when the FcR expression is increasing or, if processing can be accomplished between FcR maximum detection and ideal insemination time, reaching a maximum prior to declining to the following minimum.

Moreover, by detection of FcR expression as described herein it is contemplated that this includes, unless otherwise limited, at least one of detection of FcR mRNA, detection of FcR protein within the sperm, detection of FcR shed by sperm into their surrounding environment (e.g., vaginal fluid or in vitro media), or a combination thereof. For example, the detection of FcR expression comprises detecting the FcR shed by sperm into their surrounding environment.

Those skilled in the art may assay for and monitor other markers that correlate to fertility, for example, motility, acrosome length or morphology, appearance of the acrosomal membrane or components thereof, which appear in a punctate pattern visible through fenestrations in the plasma membrane, such pattern starting at the acrosomal rostrum and extending further down the head, expression of a cell surface molecule of sperm of the semen sample, electrostatic charge of sperm of the semen sample, and permeability of a dye by sperm or fragments thereof of sperm of the semen sample or of sperm-derived agents (such as FcR) shed into the medium surrounding the sperm. As long as the time-based assay correlates to FcR expression of sperm or shedding thereof, increased rates of pregnancy can be expected to result. Other markers may be less useful if, in contrast to the instant invention, they fail to provide sufficient time for the physician or nurse to retrieve the semen dose and perform the procedure. The predictive nature of the instant invention is thus very useful.

Thus, in one aspect not covered by the claimed invention, there is a method for determining an optimum time for utilizing a semen sample for insemination or storage comprising the steps of:
i. providing a semen sample from a male subject to be tested and incubating the sample under a controlled condition;
ii. selecting a marker that is indicative of fertility status of sperm, wherein expression of the marker correlate with fertility status of the sperm, and wherein the marker is Fc receptor (FcR) or a marker whose expression correlates with FcR expression or both;
iii. detecting the level of expression of the FcR or a marker whose expression correlates with FcR expression or both by sperm in the semen sample at a plurality of time points;
iv. determining the time point at which the sperm in the semen sample express the FcR or a marker whose expression correlates with FcR expression or both at about a maximum or a predetermined level that correlates to approaching a maximum of FcR expression of the sperm; and
v. selecting a time point where the sperm display a minimum level of expression of the Fc receptor (FcR) or a marker whose expression correlates with FcR expression or both, and thereby providing an optimum time for administering or the semen sample for insemination or freezing to provide improved pregnancy results.

In any of the aspects or embodiments described herein, the FcR comprises or is a lectin protein. In any of the aspects or embodiments, the FcR comprises or is a fucose-binding lectin protein.

In any of the aspects or embodiments described herein, the controlled condition includes incubating the semen under a constant temperature, pH or both. In any of the aspects or embodiments described herein, the controlled conditions include incubating the semen at a temperature of from about 0° C to about 37° C. In any of the aspects or embodiments described herein, the controlled conditions include incubating the semen at a temperature of from about 20° C to about 37° C. In any of the aspects or embodiments described herein, the controlled conditions include incubating the semen at a temperature of from about 22° C to about 37° C. In any of the aspects or embodiments described herein, the controlled conditions include incubating the semen at a temperature of about 37° C.

In any of the aspects or embodiments described herein, the plurality of time points of step (iii) are at least 5, 10, 15, 20, 25, 30, 25, 40, 45, 50, 55, 60 or more minutes apart. In any of the aspects or embodiments, the plurality of time points of step (iii) are at least 15, 20, 25, or 30 minutes apart.

In any of the aspects or embodiments not covered by the claimed invention, the time point of step (v) is from about 10, 15, 20, 25, 30, 35, 40, 45, 50 minutes before to about 10, 15, 20, 25, 30, 35, 40, 45, 50 minutes after the minimum level of expression of the FcR or a marker whose expression correlates with FcR expression or both.

In any aspect or embodiment not covered by the claimed invention, the step of detecting comprises detecting the amount of a ligand bound to the FcR or marker whose expression correlates with FcR expression or both. In any aspect or embodiment not covered by the claimed invention, the ligand is a small molecule label or peptide or polypeptide including a detectable marker. In any aspect or embodiment not covered by the claimed invention, the polypeptide is an anti-FcR antibody or antigen-binding fragment thereof. In any aspect or embodiment not covered by the claimed invention, the polypeptide is an anti-FcR antibody or antigen-binding fragment thereof including a detectable label. In any aspect or embodiment not covered by the claimed invention, the antibody is raised against the animal to be tested, e.g., an anti-human, anti-cow, anti-pig, or anti-horse. In any aspect or embodiment not covered by the claimed invention, the antibody is a monoclonal antibody or antigen-binding fragment thereof. In any aspect or embodiment not covered by the claimed invention, the binding of the ligand, e.g., anti-FcR antibody is detected with ELISA, western blot, quantitative immunofluorescence (QIF) assay, surface plasmon resonance, microscopy, photometer or other assay known in the art.

In any of the aspects or embodiments not covered by the claimed invention, the assay can be conducted on an aliquot of semen or an aliquot of washed sperm in a synthetic medium which, if desirable, allows one to assay sperm in wash medium and follow groups of activating sperm in the wash medium to determine the time for insemination.

In certain embodiments not covered by the claimed invention, the method further includes the step of preparing sperm in the semen sample for insemination so that the preparation is complete and insemination can occur during a period of time near when expression of the Fc receptor (FcR) or a marker whose expression correlates with FcR expression or both is at a minimum, thereby providing a preferred time for using the semen sample for insemination.

In any of the aspects or embodiments not covered by the claimed invention, step (iv) includes initiating processing of the sperm in the semen sample for administration to a female. In any of the aspects or embodiments not covered by the claimed invention, the processing includes at least one of washing the sperm, resuspending the sperm in buffer, incubating the sperm at body temperature of the host or a combination thereof.

In certain additional aspect not covered by the claimed invention, the disclosure provides methods for obtaining improved pregnancy results, the method comprising the steps of:
i. providing a semen sample from a male subject to be tested and incubating the sample under a controlled condition;
ii. selecting a marker that is indicative of fertility status of sperm, wherein expression of the marker changes with time, and wherein the marker is Fc receptor (FcR) or a marker whose expression correlates with FcR expression or both;
iii. detecting the level of expression by sperm in the semen sample of the FcR or a marker whose expression correlates with FcR expression or both at a plurality of time points;
iv. determining the time point at which the sperm in the semen sample express the FcR or a marker whose expression correlates with FcR expression or both at about a maximum or a predetermined level that correlates to approaching a maximum of FcR expression of the sperm;
v. selecting a time point which occurs before the sperm display a minimum level of expression of the Fc receptor (FcR) or a marker whose expression correlates with FcR expression or both, and thereby providing an optimum time for administering the semen sample for insemination or freezing; and
vi. inseminating the processed sperm into a female by IUI or freezing, thereby obtaining improved pregnancy results.

In any of the aspects or embodiments described herein, the FcR comprises or is a lectin protein. In any of the aspects or embodiments, the FcR comprises or is a fucose-binding lectin protein.

In any of the aspects or embodiments not covered by the claimed invention, "improved pregnancy results" can be determined or measured biochemically, e.g., by an increase in human chorionic gonadotropin levels in the female, or by detection of a fetal heartbeat, birth or combination thereof.

In any of the aspects or embodiments described herein, the controlled condition includes incubating the semen under a constant temperature, pH or both. In any of the aspects or embodiments described herein, the controlled conditions include incubating the semen at a temperature of from about 20° C to about 37° C. In any of the aspects or embodiments described herein, the controlled conditions include incubating the semen at a temperature of about 37° C.

In any of the aspects or embodiments described herein, the plurality of time points of step (iii) are at least 5, 10, 15, 20, 25, 30, 25, 40, 45, 50, 55, 60 or more minutes apart. In any of the aspects or embodiments, the plurality of time points of step (iii) are at least 15, 20, 25, or 30 minutes apart.

In any of the aspects or embodiments not covered by the claimed invention, the time point of step (vi) is from about 10, 15, 20, 25, 30, 35, 40, 45, 50 minutes before to about 10, 15, 20, 25, 30, 35, 40, 45, 50 minutes after the minimum level of expression of the FcR or a marker whose expression correlates with FcR expression or both.

In any aspect or embodiment not covered by the claimed invention, the step of detecting comprises detecting the amount of a ligand bound to the FcR or marker whose expression correlates with FcR expression or both. In any aspect or embodiment not covered by the claimed invention, the ligand is a small molecule label or peptide or polypeptide including a detectable marker. In any aspect or embodiment not covered by the claimed invention, the polypeptide an anti-FcR antibody or antigen-binding fragment thereof. In any aspect or embodiment not covered by the claimed invention, the polypeptide is an anti-FcR antibody or antigen-binding fragment thereof including a detectable label. In any aspect or embodiment not covered by the claimed invention, the antibody is raised against the animal to be tested, e.g., an anti-human, anti-cow, anti-pig, or anti-horse. In any aspect or embodiment not covered by the claimed invention, the antibody is a monoclonal antibody or antigen-binding fragment thereof. In any aspect or embodiment described herein, the binding of the ligand, e.g., anti-FcR antibody is detected with ELISA, western blot, quantitative immunofluorescence (QIF) assay, surface plasmon resonance, microscopy, photometer or other assay known in the art.

In any of the aspects or embodiments not covered by the claimed invention, the assay can be conducted on an aliquot of semen or an aliquot of washed sperm in a synthetic medium which, if desirable, allows one to assay sperm in wash medium and follow groups of activating sperm in the wash medium to determine the time for insemination.

In any of the aspects or embodiments not covered by the claimed invention, step (iv) includes initiating processing of the sperm in the semen sample for administration to a female. In any of the aspects or embodiments not covered by the claimed invention, the processing includes at least one of washing the sperm, resuspending the sperm in buffer, incubating the sperm at body temperature of the host or a combination thereof

In certain additional aspect not covered by the claimed invention, the disclosure provides methods for inseminating a female, the method comprising the steps of:
i. providing a semen sample from a male subject to be tested and incubating the sample under a controlled condition;
ii. selecting a marker that is indicative of fertility status of sperm, wherein expression of the marker changes with time, and wherein the marker is Fc receptor (FcR) or a marker whose expression correlates with FcR expression or both;
iii. detecting the level of expression by sperm in the semen sample of the FcR or a marker whose expression correlates with FcR expression or both at a plurality of time points;
iv. determining the time point at which the sperm in the semen sample express the FcR or a marker whose expression correlates with FcR expression or both at about a maximum or a predetermined level that correlates to approaching a maximum of FcR expression of the sperm;
v. selecting a time point which occurs before the sperm display a minimum level of expression of the Fc receptor (FcR) or a marker whose expression correlates with FcR expression or both, and thereby providing an optimum time for administering the semen sample for insemination; and
vi. inseminating the processed sperm into a female by IUI, thereby obtaining improved pregnancy results.

In any of the aspects or embodiments described herein, the FcR comprises or is a lectin protein. In any of the aspects or embodiments, the FcR comprises or is a fucose-binding lectin protein.

In any of the aspects or embodiments described herein, the controlled condition includes incubating the semen under a constant temperature, pH or both. In any of the aspects or embodiments described herein, the controlled conditions include incubating the semen at a temperature of from about 20° C to about 37° C. In any of the aspects or embodiments described herein, the controlled conditions include incubating the semen at a temperature of about 37° C.

In any of the aspects or embodiments described herein, the plurality of time points of step (iii) are at least 5, 10, 15, 20, 25, 30, 25, 40, 45, 50, 55, 60 or more minutes apart. In any of the aspects or embodiments, the plurality of time points of step (iii) are at least 15, 20, 25, or 30 minutes apart.

In any of the aspects or embodiments not covered by the claimed invention, the time point of step (vi) is from about 10, 15, 20, 25, 30, 35, 40, 45, 50 minutes before to about 10, 15, 20, 25, 30, 35, 40, 45, 50 minutes after the minimum level of expression of the FcR or a marker whose expression correlates with FcR expression or both.

In any aspect or embodiment not covered by the claimed invention, the step of detecting comprises detecting the amount of a ligand bound to the FcR or marker whose expression correlates with FcR expression or both. In any aspect or embodiment not covered by the claimed invention, the ligand is a small molecule label or peptide or polypeptide including a detectable marker. In any aspect or embodiment not covered by the claimed invention, the polypeptide an anti-FcR antibody or antigen-binding fragment thereof. In any aspect or embodiment not covered by the claimed invention, the polypeptide is an anti-FcR antibody or antigen-binding fragment thereof including a detectable label. In any aspect or embodiment not covered by the claimed invention, the antibody is raised against the animal to be tested, e.g., an anti-human, anti-cow, anti-pig, or anti-horse. In any aspect or embodiment not covered by the claimed invention, the antibody is a monoclonal antibody or antigen-binding fragment thereof. In any aspect or embodiment not covered by the claimed invention, the binding of the ligand, e.g., anti-FcR antibody is detected with ELISA, western blot, quantitative immunofluorescence (QIF) assay, surface plasmon resonance, microscopy, photometer or other assay known in the art.

In any of the aspects or embodiments not covered by the claimed invention, the assay can be conducted on an aliquot of semen or an aliquot of washed sperm in a synthetic medium which, if desirable, allows one to assay sperm in wash medium and follow groups of activating sperm in the wash medium to determine the time for insemination.

In any of the aspects or embodiments not covered by the claimed invention, step (iv) includes initiating processing of the sperm in the semen sample for administration to a female. In any of the aspects or embodiments not covered by the claimed invention, the processing includes at least one of washing the sperm, resuspending the sperm in buffer, incubating the sperm at body temperature of the host or a combination thereof.

As aforesaid, the marker not covered by the claimed invention can include, but is preferably not limited to, acrosome length or morphology, appearance of the acrosomal membrane or components thereof, which appear in a punctate pattern visible through fenestrations in the plasma membrane, such pattern starting at the acrosomal rostrum and extending further down the head, expression of a cell surface molecule of sperm of the semen sample, electrostatic charge of sperm of the semen sample, and permeability of a dye by sperm or fragments thereof of sperm of the semen sample or of sperm-derived agents (such as FcR) shed into the medium surrounding the sperm, expression of a cell surface molecule of sperm of the semen sample, electrostatic charge of sperm of the semen sample, and permeability of a dye by sperm or fragments thereof of sperm of the semen sample. A preferred marker is a Fc receptor on the surface of the sperm head.

In another embodiment not covered by the claimed invention, the semen sample is assayed for said marker at intervals ranging from 1, 2, 3, 4, 5, 10, 12, 15, 20, 25 and 30 minutes, which can depend on the rate of change of expression of the marker, on the time required to perform the assay, etc. In certain preferred embodiments, the semen sample is assayed at 15 or 30 minute intervals. In a further aspect not covered by the claimed invention, the assay preferably utilizes an aliquot of the semen sample or an aliquot of sperm producing results correlated to the sperm in the dose.

In another embodiment, the sperm sample is incubated at a constant temperature during the monitoring of the marker for the metabolic status.

In another embodiment not covered by the claimed invention, the marker is selected from, though preferably not limited to, a ligand, a lectin, an enzyme or a receptor, which is expressed on the surface of the sperm, or internally or both. In one embodiment not covered by the claimed invention, a ligand includes, but preferably is not limited to, a protein, a glycoprotein, a carbohydrate and a glycolipid and a lipid, including ones detected by relative membrane fluidity rather than lipid molecular structure, as reflected for example in relaxation times of fluorescence polarization measurements (as reflected by the appropriate labeled lipid probe), to distinguish different membrane regions, such as lipid rafts. In another
Embodiment not covered by the claimed invention the marker is selected from, though preferably not limited to, acrosome length, acrosome morphology, acrosome ruffling, expression of a cell surface molecule, electrostatic charge of said sperm, and permeability of sperm membrane, a lipid, cholesterol, phosphatidylserine, a sugar and a protein, an intracellular ion, and bicarbonate. As aforesaid, a preferred marker is a Fc receptor on the sperm head surface.

In any of the aspects or embodiments described herein, the marker is an FcR, including at least one of a lectin. In one embodiment, the FcR is a fucose-binding lectin.

In one embodiment not covered by the claimed invention, binding of a first ligand evokes the appearance of a secondary marker, which is detected by contacting a secondary marker with a supplemental ligand. Alternatively, assays not covered by the claimed invention can be conducted with agents that do not bind, but enter the sperm or interact in some other way (such as lipid insertion into one of the lipid bilayers in the sperm) to provoke a change with time that can be monitored, preferably for a visible change.

In any of the asepcts or embodiments, the expression of the FcR is detected in sperm, wherein the sperm are comprised in at least one of semen, vaginal fluid, media, or combination thereof.

In another embodiment not covered by the claimed invention, the permeability of a dye by said sperm or fragments thereof is assayed in an aliquot of said sperm sample by monitoring by the intensity of said dye in the sperm or fragments thereof. Sperm are known to bind seminal plasma agents onto the sperm membrane. Data suggest that sperm actually can imbibe agents from their surrounding medium. Therefore, fragments of sperm should be construed to include agents sperm acquire as cargo, not just sperm as formed in the testis or epididymis.

In another aspect the description not covered by the claimed invention provides a method of monitoring the expression of a cell surface marker, wherein and the time point selected to process the semen sample is determined with respect to an earlier time point when the sample maximally expresses the marker. In certain embodiments not covered by the claimed invention, the time point selected to process the semen sample is determined with respect to the first determined increase in expression of the cell surface marker as compared to a control or baseline value. In certain embodiments not covered by the claimed invention, the first uptick is a relative increase in expression of about 1, 2, 3, 4, 5, 6, 7, 8, 9, 10, 11, 12, 13, 14, 15 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100% or more (including all values in between).

In another embodiment not covered by the claimed invention, the expression of a cell surface marker is monitored, and the time point selected to perform the IUI procedure with the washed portion of the semen sample is determined with respect to the earlier time point when expression of said marker in said sample has decreased relative to a peak expression and subsequently begins to increase from a minimum in the expression.

In one embodiment not covered by the claimed invention, the assay is based on the percentage of sperm in the semen sample having a specified marker. In one aspect, the specified marker is a biochemical marker, which is optionally present on the cell surface. Regardless, the marker reflects or is indicative of the metabolic fertility status of the sperm. The marker is not limited to a sperm specific marker.

In one embodiment not covered by the claimed invention, the assay encompasses determining the percentage of sperm in the semen sample having the marker that reflects the metabolic fertility status of the sperm. The assay comprises the steps of a) removing an aliquot from the semen sample; b) contacting the aliquot with a first ligand to said marker; c) detecting binding of said ligand by said sperm; and d) determining the percentage of sperm in said aliquot which binds the ligand. In an alternative aspect of this embodiment, step d) can be replaced by the step of assessing the level of the marker by a detectable label which binds the marker or the ligand either directly or indirectly.

In another embodiment not covered by the claimed invention, the assay encompasses determining the concentration of said marker detected in sperm of said semen sample comprising: a) removing an aliquot from the semen sample; b) contacting the aliquot with a first ligand to said marker; c) detecting binding of said ligand by said sperm; and d) determining the amount of marker expressed by sperm in said aliquot by quantitating the binding of the marker by the ligand; thereby determining the concentration of said marker detected in sperm of said semen sample.

In certain embodiments not covered by the claimed invention, the ligand is labeled with a detectable label, preferably a visible label. In certain embodiments not covered by the claimed invention, the ligand is a labeled antibody; e.g., a labeled anti-FcR antibody or a labeled antibody which binds to an anti-FcR antibody.

In another embodiment of this assay not covered by the claimed invention, detecting the binding of the ligand by the sperm includes detecting label bound directly or indirectly to the sperm, or fragments of the sperm. In one aspect not covered by the claimed invention, a sperm fragment is either associated or disassociated from intact sperm. In another aspect not covered by the claimed invention, detecting the binding of the ligand by the sperm includes detecting label bound to an agent that was initially present in semen, then associated with sperm and then dissociates from sperm or is shed from sperm.

In another embodiment not covered by the claimed invention, detecting the binding of the ligand by the sperm encompasses contacting the sperm in the aliquot with a second ligand which binds to the first ligand.

In another embodiment not covered by the claimed invention, the first ligand and/or the second ligand is an antibody. The antibody can be either polyclonal or monoclonal antibodies, and can comprise one or more labels.

In another embodiment of the methods and assays not covered by the claimed invention, an indicator of the metabolic fertility status of the sperm is a biochemical marker. The biochemical indicator includes, but is not limited to, one or more of the following biochemical indicators: a cell surface molecule, an enzyme and a receptor. Indicators of the status of the sperm can also use the ability of sperm to imbibe a dye, bead, or other agent from the medium surrounding the sperm, to produce a measurable signal by known detection methods.

The present inventor has also unexpectedly discovered that FcR additive can protect IUIs from men with low sperm counts (oligozoosperma). Without being bound by any particular theory, it is believed that these ejaculates do not have sufficient FcR in the media due to low cell numbers. One of the detrimental effects of low cell number, deficient amount of FcR is shed into the media, can be ameliorated via the administration of an effective amount of an exogenous FcR. Thus, in an additional aspect not covered by the claimed invention, the present description provides methods of improving fertilization of an egg by sperm, e.g., an animal such as a mammal, including a pig, cow, or human, comprising administering to a female an effective amount of an Fc receptor. The method can be used in conjunction with the processing methods as described herein or alone. In certain embodiments not covered by the claimed invention, Fc receptor is administered directly to the oviduct or uterus of a female, e.g., female mammal such as a pig, cow or human. In certain embodiments, the FcR is co-administered with semen or sperm, e.g., semen or sperm processed according to the methods described herein. In any of the aspects or embodiments described herein, an effective amount of FcR can be formulated in a composition comprising one or more pharmaceutically acceptable carriers and/or adjuvants before administration or before or after combining with semen or sperm, e.g., semen or sperm processed according to methods described herein. Acceptable carriers or diluents for therapeutic use are well known in the pharmaceutical art, and are described, for example, in Remington's Pharmaceutical Sciences, Mack Publishing Co. (A. R. Gennaro edit. 1985).
When impregnating humans, it is preferred to collect shed FcR when washing sperm to provide sperm for insemination and, then adding the shed FcR back to the washed sperm for insemination.

As such, in certain aspects, the description provides a composition comprising an effective amount of an FcR in combination with a pharmaceutically acceptable carrier. In certain embodiments, an effective amount is a dosage of from 0.01 ng/kg to about 1000mg/kg. In certain embodiments, the effective amount is from about 1 pg to about 1kg, including all values in between, including from 1, 10, 100pg; 1, 10, 100 ng; 1, 10, 100 ug; 1, 10, 100mg; 1, 10, 100g; to 1kg. In certain embodiments of the compositions or methods described herein, the FcR is administered at a concentration of about 0.01 ng/ml to about 1000mg/ml, including, all values in between. In certain embodiments, the 1 mg of FcR is administered.

As used herein, the terms "co-administration" and "co-administering" refer to both concurrent administration (administration of two or more agents, e.g., sperm and added FcR, at the same time) and time varied administration (administration of one or more agents, e.g., sperm and added FcR, at a time different from that of the administration of an additional agent or agents), as long as the agents are present in the female to some extent, preferably at effective amounts, at the same time.

In accordance with this aspect, one or more FcRs can be formulated into a composition and/or administered as described herein, including at least one of CD16A; CD16a antigen; CD16B; Fc fragment of IgG low affinity IIIa receptor; Fc fragment of IgG, low affinity III, receptor for (CD16); Fc fragment of IgG, low affinity IIIa, receptor (CD16a); Fc fragment of IgG, low affinity IIIb, receptor (CD16b); Fc gamma Receptor 3; Fc gamma Receptor III; Fc gamma receptor III-A; Fc gamma receptor IIIa; Fc gamma receptor IIIb; Fc-gamma receptor III-2 (CD 16); Fc-gamma receptor IIIb (CD 16); Fc-gamma receptor IIIb (CD16); fc-gamma RIII; Fc-gamma Rill-alpha; fc-gamma RIII-beta; Fc-gamma RIIIa; fc-gamma RIIIb; FcgammaRIIIA; FCGR3; FCGR3A; FCGR3B; FCGRIII; FCR-10; FCRIII; FCRIIIA; igG Fc receptor III-1; igG Fc receptor III-2; immunoglobulin G Fc receptor III; neutrophil-specific antigen NA; RP11-5K23.1; protein A; protein G; or a combination thereof including either a mixture or a recombinant molecule containing elements of both protein A and protein G. There are a number of FcR that are commercially available, including CD16 (R&D Systems).

In accord with the present invention but not covered by the claimed invention, the time-based assay can also be used as a diagnostic assay for male infertility. It also can be used to prepare frozen or frozen-thawed doses of sperm for later use for IUI to provide sperm in the right fertility state for more successful IUI pregnancy results.

### Examples not covered by the claimed invention

### Example 1. PREFERRED SPERM WASH PROCEDURES FOR IUI

The objective is to prepare motile spermatozoa using density gradient separation and/or spin down sperm washing techniques for Intra-uterine Insemination (IUI).

In the density gradient separation technique described herein, the discontinuous density gradient centrifugation is used to isolate and purify the motile sperm in order to obtain a sample free of seminal plasma agents that can cause cramping and containing the a good concentration of high quality sperm to be use for intrauterine insemination (IUI). Different concentrations of fluid are layered in a test tube in an ascending order of density (heaviest layer at the bottom). When a semen sample is placed upon the upper-most layer and centrifuged, any debris, round cells, non-motile and poor quality sperm remain in the top layers. Only the motile sperm are able to get through to the bottom layer and are then concentrated for use in IUI.

The spin down technique in IUI also serves to enhance final sperm concentration and motility. In this technique, liquefied semen sample is mixed with sperm wash medium, centrifuged and the supernatant discarded and re-suspended again with equal amount of wash medium.

### Preparation of Density Gradient Sperm Wash Media

Bring all reagents to room temperature. Place all equipment and materials required for preparation of the Sperm Wash Media inside the biological safety cabinet (BSC).

Prepare required number of sterile conical tubes. Label tubes with assigned number of reagent bottle (numbered by order received) and "80%" or "W" depending on whether gradient or wash media is being aliquotted. The entire reagent bottle can be aliquotted and tubes stored at 4°C until use. If only partially aliquotted, store the opened reagent bottle at 4°C. Suitable gradient and wash media are any such media commonly used in ART facilities. Non-limiting examples include PureSperm (Nidacon catalog #PSB-100), Spermcare upper layer (45%) and Spermcare lower layer (90%), (InVitroCare catalog #2221-100 and #2222-100, respectively), Spermwash (InVitroCare catalog # 2003), Sperm Preparation Medium (Origio, catalog # 10690010A).

*Prepare the 1 Layer Sperm Wash Medium (80%):* pipette 2 mL of 80% gradient medium (for example, PureSperm 80 (Nidacon, catalog # P580-100) into sterile conical tubes.

*Prepare the Sperm Wash Medium:* pipette 4 mL of sperm wash medium (for example, PureSperm Wash (Nidacon, catalog #PSW-100) into sterile conical tubes.

At the start of the work day, put the prepared density gradient and sperm wash media in the 37°C incubator until use. According to the number of procedures, put 2 gradient tubes and 1 wash media tube per IUI.

### 1. Layer Density Gradient Separation Technique

### Pre-wash Semen Analysis and Sperm Wash Proper

*Prepare 2 Makler*^{®} *Counting Chambers for pre-wash semen analysis using 5 µL semen sample.* Using a sterile Pasteur pipette, gently dispense a maximum of 2 mL liquefied semen sample onto the top of the gradient medium.

*Procedural note: Use additional gradient medium if semen sample volume is > 2 mL.*

Note: Prior to start of any sperm wash procedures, check semen sample visually. If sample does not liquefy sufficiently at a given time (<60 min from sample production), add sperm wash medium until liquefaction is achieved.

Visualize sample to establish presence of motile sperm. Centrifuge for 20 minutes at 280xg. Perform pre-wash semen analysis using the prepared counting chambers above.

*Do not use the brake to stop the centrifuge.*

Remove the supernatant down to the pellet using sterile Pasteur pipette. If there is no visible pellet, aspirate all supernatant, leaving 300ul of fluid.

Prepare and label another sterile conical tube, pour 3 mL of sperm wash medium; from this solution use 0.3 mL to re-suspend the pellet. Transfer the re-suspended pellet to this tube using a sterile Pasteur pipette.

Centrifuge for 10 minutes at 420xg.

Remove the supernatant down to the pellet using sterile Pasteur pipette.

Re-suspend the pellet with 0.5 mL of sperm wash medium.

*At this time, prepare 2 Makler*^{®} *Counting Chambers for post-wash semen analysis using 5 µL semen sample.*

Post-wash Semen Analysis

Perform post-wash semen analysis using the prepared counting chamber above.

Thawing of frozen semen samples

Frozen straw/s - place straw/s first in a conical tube prior to thawing in water bath (37°C) for 5 minutes; transfer thawed semen sample in a sterile conical tube. Mix.

Cryotubes/vials - place cryotubes/vials first in a cryotube floating rack prior to thawing in water bath (37°C) for 5 minutes; transfer thawed semen sample in a sterile conical tube. Mix.

Pre-wash Semen Analysis and Sperm Wash

Obtain the thawed semen sample from the water bath.

*At this time, prepare 2 Makler*^{®} *Counting Chambers for pre-wash semen analysis using 5 µL semen sample.*

Perform pre-wash semen analysis using the prepared counting chamber above.

Add 3 mL of sperm wash medium to the thawed sample, carefully dispensing the wash medium on the side of the conical tube.

Centrifuge for 10 mins at 420g.

Remove the supernatant down to the pellet using sterile transfer pipette.

Re-suspend the pellet with 0.5 mL of sperm wash medium.

Place the washed sperm suspension in the incubator (37°C) until insemination.

Post-wash Semen Analysis

Perform post-wash semen analysis using the prepared counting chamber above.

Reference Ranges/Expected Values

| | **Lower Reference Limits (W.H.O. 2010)** |
|---|---|
| Volume (mL) | ≥ 1.5 mL |
| Count (M/mL) | ≥ 15 M/mL |
| Motility (%) | ≥ 40 % |
| Agglutination | < 10 % |
| WBCs | < 1.0 M/mL |

### Example 2.

### Fc RECEPTOR ASSAY

The assay is performed directly on 2.5-10 µl aliquots of raw semen, thawed washed semen in synthetic medium or washed semen in synthetic medium. A larger amount of raw semen may be used for oligozoospermic samples, however, the preferred method of cell counting for oligozoospermic samples, where possible, is to resuspend the cells in a smaller buffer volume for cytometric analysis, as low as 100 µl, and to collect events on what corresponds to the fastest flow rate on an BD Accuri c6 cytometer. Semen is collected into a collection cup.

Before running this assay, be sure (1) that semen is collected and incubated as described in Working Example 1 to minimize process failures and (2) that assay reagents are at ambient temperature before use (22-26°C). GREEN 1, RED 2 and BLUE 3 are discussed above.

PREPARE ASSAY MIXTURE (not premixed)
i. Into 1.5ml tube, pipet the following IMMEDIATELY before use and in the order directed below.
ii. 100 µl GREEN
iii. 20 µl RED 2, mix
iv. 10 µl neat semen, mix
v. 5 µl BLUE 3, mix.
vi. You will repeat this assay at 30min intervals, but ideally 15 min intervals. Keep reagents at ambient at all times during assay run.

PREPARE ASSAY MIXTURE (Pre-mixed and pre-aliquotted reagents)
i. Into 1.5ml tube, pipet the following IMMEDIATELY before use and in the order directed below, or optionally pre-aliquotte 120 µl GREEN 1/ RED 2 mixture into 1.5ml tubes.
ii. 120 µl GREEN 1/ RED 2 mixture (mixed 5:1 v/v)
iii. 10 µl neat semen, mix
iv. 5 µl BLUE 3, mix.
v. You will repeat this assay at 30min intervals, but ideally 15 min intervals. Keep reagents at ambient at all times during assay run.

### INCUBATE

Incubate tube for 20 minutes at ambient temperature. (A 15 minute incubation is possible but the risk of artifact increases if the assay is shortened excessively.)

### WASH

i. Add 1ml BUFFER at ambient temperature (PBS buffer (8 g NaCl; 0.2 g KCl; 1.44 g Na₂HPO₄ • 7H2O; 0.24 g KH₂PO₄; H₂O to 1 liter. PH 7.2))
ii. Microfuge 30 seconds at 2000 x g (about 6,000rpm in microfuge).
iii. Carefully remove supernatant with 1ml pipette. Leave small amount of fluid (typically about 40-70 µl, depending on firmness of sperm pellet) to avoid disturbing pellet.

### SCORE

Add ~300ul BUFFER to cell pellet and mix gently to resuspend. Resuspension volume for scoring is related to sperm concentration: oligozoospermic samples require smaller resuspension volumes, as low as 100 µl, or counting times will be lengthened to analyze the required number of events. Where cell number is low, it is preferred to first increase flow rate on the cytometer. If this permits acquisition of 5,000 events within about 60 seconds, it is preferable. If sperm counts are too low to enable this, volume reduction at resuspension is needed. Alternatively, if low sperm count is suspected to become a problem prior to starting the assay run on an ejaculate, assay 10ul aliquots, not less, at all time points. It is preferred to use the same volume of sample in each multipoint assay from a particular ejaculate in order to provide best results.

Place tube containing resuspended cells onto cytometer SIP tube and analyze on a calibrated cytometer using the "Cytometer Scoring" template (see Cytometer Scoring SOP for further details).

### ANALYZE DIAGNOSTIC RESULTS AND DETERMINE PROCESSING TIME FOR OPTIMAL FERTILITY

### Diagnostic analysis

FcR initial status--(NOTE: this diagnostic can be run ONLY when one or more coagulated samples are tested immediately post-collection. Liquified samples may have normal cohorts already maturing, resulting in scores that are high but nonetheless normal.) Determine the percentage of positive sperm at the first time point. If it exceeds 35%, the collection shows abnormal premature deployment of FcR. This is associated with infertility in livestock IUI. Even an initial result of 15% on a coagulated sample raises the index of suspicion for male-factor infertility.

FcR kinetics-plot FcR % positive against time. See FIGs. 9A(1) - 9F(2). Normal kinetics over a 2.5h period involve maturation of more than one group of sperm, with usual periodicity of 1.0-2h (usually 1h), although some collections show groups maturing at shorter intervals. In humans, cattle and pigs, swim up yield, a known fertility marker in cattle IUI, is maximal in humans about 0.5h after peak FcR positivity, making FcR kinetics a predictive assay. Ejaculates showing only a single cohort or group of sperm activating by FcR have been found in some cases to also have other abnormalities. Single cohort ejaculates are not seen in livestock. These results raise the index of suspicion that single-cohort ejaculates may have male-factor issues in pre-ART (IUI) that could compromise pregnancy outcome.

Improving freezing damage resistance-it is known that increasing fluidity of membranes correlates with improved freezability. Because the methods as described herein measure FcR receptor expression, reflective of membrane changes resulting in increased fluidity, the assay can be used to optimize viability of samples that are to be frozen, by allowing freezing at a state that minimizes the cell damage that occurs upon thawing. As sperm processing conditions for freezing differ between laboratories, it is best for each laboratory to calibrate the sperm state that is best, when combined with their specific process, at improving post-thaw viability. This can be done by running the assay and processing/ freezing aliquots at the start of each assay time point. The sperm state, identified by assay, that produces the preferred post-thaw attributes (for example, highest viability on post-thaw analysis, high swim up yield, best result in sperm penetration test that measures binding to and penetration of egg by sperm, or other preferred indicator) is the state most suitable for dose freezing by the procedures in that specific laboratory. Note that the maximal attainment of the preferred post-thaw attribute may not occur immediately after thawing, but only after some time has elapsed. This is due to the possible differences in time of attainment of the preferred state for freezability of sperm versus attainment of the preferred post-thaw attribute.

### Treatment with state-adjusted sperm for IUI

Plot percentage of positive cells. The percentage of cells positive for the Fc receptor will usually begin to increase in the timeframe of 30min-2.5 h post-collection for coagulated samples, and may already be elevated at first assay point for post-liquefaction samples with early-maturing sperm groups, confounding interpretation of the first FcR peak. Time zero = time of assay inception.

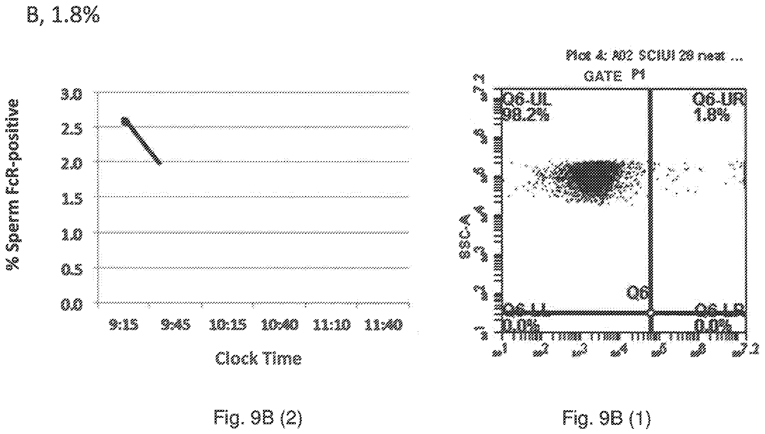

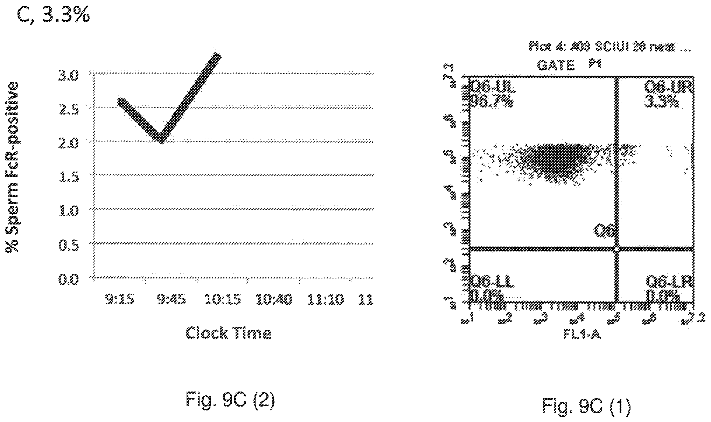

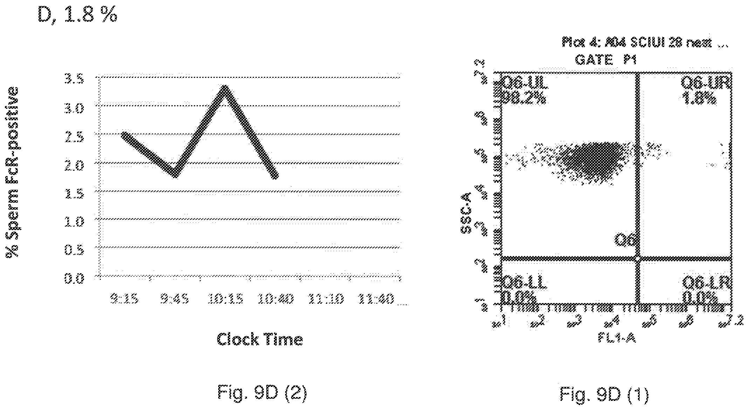

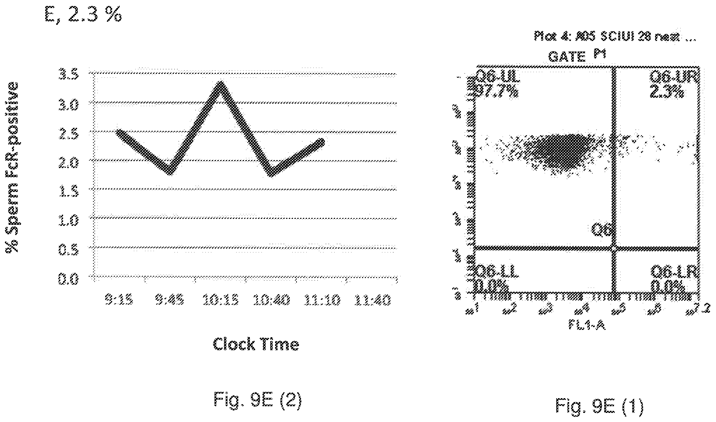

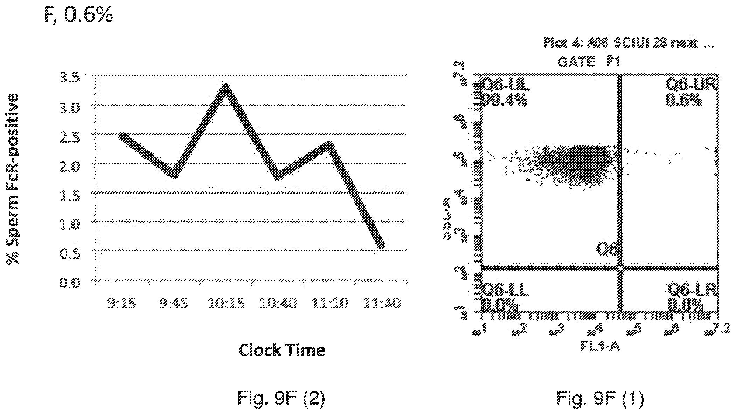

Graph points, looking for an increase between two points of at least 30% for approximately 30 min intervals. The increase between three points should be at least 30% when tested at approximately 15 min intervals. In certain embodiments, the interval is approximately 1, 2, 3, 4, 5, 6, 7, 8, 9, 10, 11, 12, 13, 14, 15, 16, 17, 18, 19, 20, 21, 22, 23, 24, 25, 2,6 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 7,9 80, 81, 82, 83, 84, 85, 86, 87, 88, 89, 90 minutes or more. When such an increase in positivity of cells is seen, perform procedure of IUI insemination with the portion of the sample prepared for this purpose in the window from assay end (when reading becomes available) to 20 minutes after that assay end point was obtained. See the example in Figure 9. The ideal window of time falls within approximately 30-60 minutes, and processing time may necessitate insemination at approximately 45-60 min, from the time of FcR peak maximum as determined from assay start time (not end time). Where possible, however, it is preferred that sperm be introduced into the uterus by IUI at a time when their expression of FcR is falling and reaching a minimum, as opposed to passing the minimum and climbing.

See FIGs. 9A(1) - 9 F(2) for an actual sample example. Note that smaller increases in % positive FcR may still be associated with favorable outcomes, but are not preferred for sperm fertility state adjustment.

FIGs. 9A(1) - 9 F(2) show an example for identification of FcR peak and desired IUI time. Figure designated 9B (2) shows a decreasing population of FcR positive sperm, which often precedes a time point showing a state of increase, from which timing of sperm IUI should occur. Thus, the figures9B (2) and 9C (2) are the ones that would signify to the clinic that the critical sperm state is being approached and then attained. For 30min sampling intervals shown here, the FcR high percent positivity at 10.15 clock time indicates that IUI should be performed between 10.30-11.00 for a window of +/- 15min, or 10.30-11.05 for a window of +/- 20min. This is practice means between the end of the assay, when this time point's data are available, which for 30min assay intervals is 10.40, up to 11.00 but preferentially close to 10.40. The window of time would span 10.30-11.00 ideally, or maximally 10.25-11.05 if allowing +/-20min from time of FcR minimum to insemination. The anticipatory signals the assay provides are important because the clinic will not see the entire curve during sperm preparation, only the region necessary to adjust sperm state to the IUI-compatible state. Obviously, more frequent sampling is desirable both for better curve definition and for enabling use of a larger portion of the window, containing the preferred sperm state, for performance of the IUI procedure. But the correctness of the IUI window is confirmed by seeing a sharp decrease in % FcR positive at the time point where assay was started at 10.45am, followed by another increase in the assay run that started at 11.15.

### CYTOMETER SCORING

Before scoring this assay, be sure that semen is collected and incubated exactly as set forth above and that the assay is run exactly as described above, to minimize process failures.

### START EQUIPMENT

i. Turn on computer
ii. Turn on cytometer (BD/Accuri, San Jose, CA)
iii. If needed, empty waste bottle and fill sheath bottle with DI water

### OPEN TEMPLATE AND NAME FILE

i. Click Assay 1 Template;
ii. Select File > Save CFlow file as...;
iii. Name file by date by typing date code under File Name;
iv. Click Save.

### COLLECT DATA

i. Under the red Collect tab, click on desired cytometer grid (1A is for first sample, first donor, 1B is for second sample, first donor. 2A is for first sample, second donor, etc.);
ii. Next to cytometer grid (e.g., A01) type sample information and assay time (time when collection is assayed);
iii. Check that stoplight shows green color. Load sample onto SIP tube on cytometer and pull out plastic tube support underneath tube;
iv. Click Run;
v. After data are acquired, adjust gate on density plot so that the percentage of positive sperm (the population on the right) can be determined. Record this number. Remove sample from SIP tube;
vi. For the next sample, repeat process above starting with step 3a;
vii. After the samples for that assay time are finished, click Backflush, wait for stoplight to show green, then click Unclog.

### CLEAN AND SHUT DOWN EQUIPMENT

i. Between every 5-10 samples, place waste cup under SIP, then select Backflush or Unclog.
ii. At the end of the day, place a tube of cleaning fluid on the cytometer, select well H11 and click Run. Run for 2 minutes. Then place a tube of water on the cytometer, select well H11 and click Run. Run for 2 minutes. Allow water to clean system for at least 2 minutes. (Note, due to some movement of the negative pool on rare samples, it is preferred to set the positive fluorescence gate with some space between it and the negative pool. This allows accommodation of some negative pool movement without causing negative events to gate into the positive pool. It is not preferred to re-gate a time point with profound abnormal negative pool movement, but sometimes this is necessary due to degree of abnormality.)

### Turn off cytometer, then turn off computer;

i. Follow all instructions for operation, cleaning and preventive maintenance as detailed in the manufacturer's instrument manual.

### Example 3.

### INCREASING SIGNAL OF ASSAY

The human fertility clinic is faced with samples that are oligozoospermic (low sperm count) or otherwise abnormal. For these samples, it may be desirable to increase the signal intensity of the assay. Two methods for doing this are described herein: (1) use of washed cells and (2) use of a different assay diluent. Examples of wash medium include Sperm Wash Medium sold by a number of vendors, for example, Sperm wash medium (InvitroCare Cat #2005) or Sperm Washing Medium (Irvine Scientific Catalog ID 9983). Examples of a different assay diluent include buffer which can be substituted for Green 1. Sample for which buffer is suggested are shown in the protocol below. While sample type and diluent type can be changed, preferred embodiments are presented in the protocol below. While some samples may show good performance in wash medium, some ejaculates fail to demonstrate cycling in wash medium and this behavior is associated with failure to achieve pregnancy, as is true for cycling failure when sperm are assayed in semen.

### PREPARE ASSAY MIXTURE

i. Into 1.5ml tube, pipette the following IMMEDIATELY before use and in the order directed below.
ii. 100 µl GREEN 1 (or Buffer if using neat semen)
iii. 20 µl RED 2, mix
iv. 10 µl washed cells --if at about the same concentration as in neat ejaculate, otherwise, please adjust volume analyzed or amount of reagents used, mix or 10 µl neat semen (if using Buffer), mix
v. 5 µl BLUE 3, mix.
vi. Repeat this assay at least at 30min intervals, but ideally 15 min or less intervals. Keep reagents at ambient at all times during assay run.

### INCUBATE

Incubate tube for 20 minutes at ambient temperature. (A 15 minute incubation is possible but the risk of artifact increases if the assay is shortened excessively.)

### WASH

i. Add 1ml BUFFER at ambient temperature; mix by inversion
ii. Microfuge 30 seconds at 2000 x g (about 6,000rpm in microfuge);
iii. Carefully remove supernatant with 1ml pipette. Leave small amount of fluid to avoid disturbing pellet, depending on how firm the pellet is, this can typically range from 30-80ul.

### SCORE

i. Add ~300 µl BUFFER to cell pellet and mix gently to resuspend. Resuspension volume for scoring is related to sperm concentration: oligozoospermic samples require smaller resuspension volumes, as low as 100 µl, or counting times will be lengthened to analyze the required number of events.
ii. Place tube containing resuspended cells onto cytometer SIP tube and analyze on a calibrated cytometer.

### ANALYZE DIAGNOSTIC RESULTS AND DETERMINE PROCESSING TIME FOR OPTIMAL FERTILITY

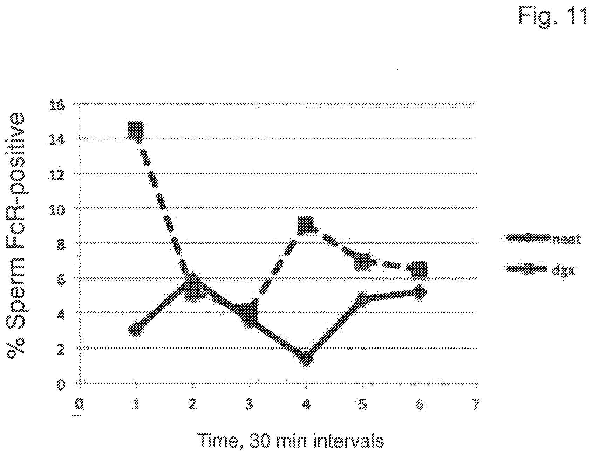

FIG. 11 shows an example of comparison of washed sperm and neat semen assays.

Solid line shows neat semen. Dashed line shows sperm from same ejaculate washed by density gradient centrifugation (dgx) before assay. Average % FcR positive for neat semen is 4.0 and for washed sperm is 7.7, over the course of the assay which was run at 30 minute intervals. The ratio of cohort positivity for the full peaks at time point 2 for neat semen and time point 4 for washed sperm is 2.1 (9.1% washed / 4.4% neat), meaning cohort signal in washed sperm is double that in neat semen. As the samples are from the same ejaculate, so cohort size comparison is easier, but not absolute. However, it is clear that the neat sperm cohorts peak at a lower % positive than the washed sperm cohort (a partial one, at 14.5% positive, is seen at the first time point on the washed sperm). Please note, due to the washing conditions it is difficult as mentioned to run identically-timed assays. These data are for comparison of degree of positivity, not for comparing washed and neat assays by time point as correspondence is loose.

### Example 4.

### SEMEN DOSE FREEZING

In cryobank procedures, to provide semen resistant to freeze-thaw damage, perform the time-based assay in accord with the present invention as follows.

### OBTAIN ALIQUOT OF COLLECTION FOR ASSAY

Run assay on a 10 µl sample immediately upon receipt in the laboratory. Use this as a diagnostic for sperm use in pre-ART. If the percentage of FcR positive sperm at the first time point exceeds 35%, it is possible to run the procedure but the collection should be flagged as potentially abnormal. It may not produce pregnancy efficiently in pre-ART. (NOTE: ensure reagents are at ambient temperature for use to avoid production of assay artifacts.)

If it is not possible to collect an immediate sample, but only post-liquefaction samples, remove 50 µl aliquot within 30 minutes of sample receipt and keep at 37°C. If the laboratory prefers to use ambient storage, this is compatible with the test but please note this deviation in the processing records.

### ASSAY

Follow instructions in the Example 2 for evaluating sperm cells and carrying out further processing.

All patents, patent applications, and published references cited herein are hereby incorporated by reference in their entirety. The disclosure set forth herein has been particularly shown and described with references to preferred embodiments thereof, it will be understood by those skilled in the art that various changes in form and details may be made therein without departing from the scope encompassed by the appended claims.

### Example 5.

### Administration of FcR

Aliquots of FcR were weighed out on a microbalance into 1.7ml microcentrifuge tubes, cap and store in freezer (FcR Pierce Protein A/G Recombinant, catalogue number PRO-646, lot QG221923 from E. coli). For extender, BioXcell received 12/11/16, made by IMV, expiry 2016-03, was used (BioXcell 5032401).

The mixture was made using BioXcell by adding 2ml concentrate from IMV into 8ml water, mix well. Take into this 10ml of extender, 20mg Pr A/G for prep of 0.5ml dose having 1mg/dose at AI. These were put into 11ml of the diluted (AI concentration) BioXcell at a concentration of about 2mg/ml (a dose of 0.5ml produces an FcR dose of 1mg per AI [artificial insemination, in this case cattle IUI] when inserted into female). This was followed by semen.

### Example 6

### Washed Sperm

IUI procedures involve washing sperm to prepare for insemination into female. Washed sperm samples were prepared and analyzed using the Kinetix^{™} procedure as described in Example 2 above, the same as for analyzing neat semen.

One data set included a patient for washed sperm who received 2 IUIs on successive days, with one compliant and one non-compliant ejaculate. This data set was coded as WIUI 10 and WIUI 13, and was omitted from tables below because it could not be confirmed as to which sample resulted in pregnancy. Compliant means insemination within window about the FcR minimum, of +/- 20min from minimum. As can be seen in the tables, Year 1 trials were exactly reproduced during Year 2 trials: all pregnancies in +/- 15-20min window of FcR minimum.

The Tables below show results of trials indicating pregnancies achieved when sperm ejaculate samples were compliant or non-compliant. Scoring was by curve shape, with the understanding that ideal interpretation of a curve, f(x), is according to the rate of change of the assay function with time, that is, d(x)/dt, to identify curve maxima, minima, and inflection points where d(x)/dt changes sign (from positive to negative or negative to positive) or changes most rapidly. Changes in sign occur at maxima and mimina points, while most rapid rate of change occurs at inflection points on the rising and falling sides of the sine curve. On an ideal sine curve, we would prefer to predict FcR minimum through detection of d(x)/dt= 1 (the inflection point on the rising side of the sine curve, confirm FcR increase by d(x)dt=0, and IUI at d(x)/dt within +/-15min of the minimum where d(x)dt = 0, and has changed from negative to positive.

Year 1 Trial, sperm in semen.

The lab sample of an ejaculate was assayed at 30 min intervals by the invention, while sperm in the remaining ejaculate were processed for insemination. The assay time included the time at which insemination of the patient occurred. Kinetix-compliant IUI was defined as the insemination procedure occurring within +/- 20min of the nearest FcR minimum by assay. Kinetix non-compliant IUI was defined as insemination occurring outside of the +/-20min window of the nearest FcR minimum.

| # Patients | Kinetix Status, ( compliant = IUI +/-20min of FcR min) | # Preg | % Preg |
|---|---|---|---|
| 29 | Total IUI all types | 6 | 6/29 = 21% |
| 15 | Kinetix -compliant | 6 | 6/15 = 40% |
| 14 | Kinetix non-compliant | 0 | 0/14 = 0% |

Year 2 Trial, sperm in wash medium. Sperm in wash medium were assayed in duplicate 2.5 ul, samples with half the normal assay reagents volumes, at 30min intervals by the invention, and replicate points were averaged to create the curve. The assay time included the time at which insemination of the patient occurred. Kinetix-compliant IUI was defined as the insemination procedure occurring within +/- 20min of the nearest FcR minimum by assay. Kinetix non-compliant IUI was defined as insemination occurring outside of the +/- 20min window of the nearest FcR minimum.

| # Patients | Kinetix Status, ( compliant = IUI +/-20min of FcR min) | # Preg | % Preg |
|---|---|---|---|
| 16 | Total IUI all types | 2 | 2/16 = 13% |
| 10 | Kinetix -compliant | 2 | 2/10 = 20% |
| 6 | Kinetix non-compliant | 0 | 0/6 = 0% |

Aggregation of the sperm samples from both years gives:

| # Patients | Kinetix Status, ( compliant = IUI +/-20min of FcR min) | # Preg | % Preg |
|---|---|---|---|
| 45 | Total IUI all types | 8 | 8/45 = 18% |
| 25 | Kinetix -compliant | 8 | 8/25 = 32% |
| 20 | Kinetix non-compliant | 0 | 0/20 = 0% |

The two-sample test for equality of proportions can now be performed on this 2x2 table. Note that because some of the cells in this table have low counts (e.g., the number of pregnancies in both Kinetix compliance groups are less than 10), we apply Yates' continuity correction to the data. This continuity correction corrects for the low cell counts, and results in a conservative statistical test. That is, if the two proportions of pregnancies are truly different from each other, a test incorporating the Yates continuity correction may under-estimate this difference.

This test for equality of proportions has 1 degree of freedom. The resulting Chi-square statistic is 234 = 5.7485, and the corresponding p-value is % = 0.008251. Since this p-value is less than the significance level of > = 0.05, we reject the null hypothesis, and conclude that the proportion of pregnancies observed among Kinetix assay compliant patients is statistically significantly greater than the proportion of pregnancies observed among the non-compliant patients. With 95% confidence, the data are consistent with pregnancy achievement proportions between 0.122 and 1.00 for Kinetix assay compliance patients.

### Example 7

### Assay Cycling Rate Correlates to Pregnancy Rate

Semen samples were assayed as described in Example 2 above. Graphs are neat semen assays from ejaculates, showing % Kinetix positive on Y axis and time on X-axis. Group I has no minimum; Group II has one (1) minimum; Group III has two (2) minima.

### Relationship of Ejaculate Kinetix Cycling Rate and IUI Pregnancy Achievement

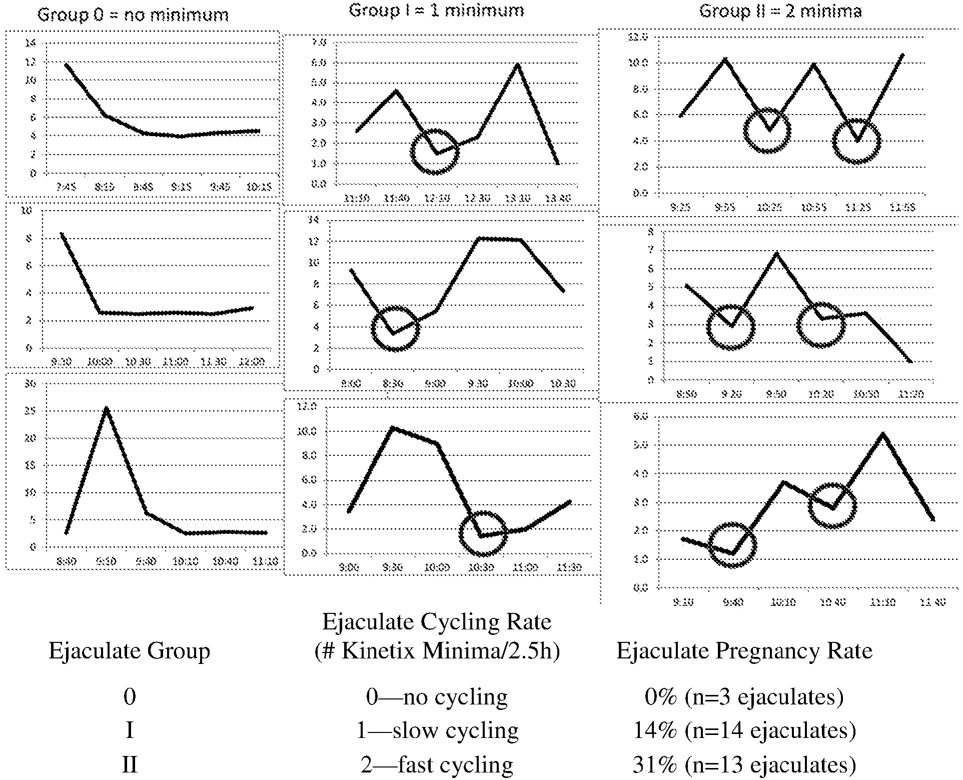

Neat ejaculates were assayed as described and IUI procedures for sperm processing and insemination were done in blinded fashion, with inseminations occurring randomly throughout the permitted procedure window. The percentage of ejaculates showing no, slow or fast cycling were evaluated for pregnancy outcome.

These data illustrate use of cycling for diagnostic purpose, for example by cryobanks, by IUI clinics who want the couple back again if the first ejaculate produces no Kinetix^{™} assay minimum, or one minimum when they may want to try for a 2-minimum ejaculate the next day. IUI procedures 24h apart is an established practiced in the clinic for certain patients.

Thus, clinics and cryobanks can use the Kinetix^{™} assay-not just as manufacturing process control to make semen doses for IUI that are of uniform sperm state-but as a diagnostic. Fast-cycling ejaculates will be more fertile in IUI. If a male patient produces a Group I or Group II ejaculate, a physician may wish to call the couple back to process another ejaculate 24h later, so that chances of pregnancy achievement are increased (when first ejaculate was suboptimal). A cryobank may desire to store only rapid cycling ejaculates for IUI-and reject the Group 0 noncyclers. The Kinetix^{™} assay is run exactly as disclosed in Example 2 above, on neat semen. Exact processes currently in use at clinic or cryobank would be used for processing the semen for IUI. The only change would be to identify an ejaculate's Kinetix^{™} cycling rate to determine sperm dose IUI quality or to determine patient stratification into single IUI or repeat IUI procedures.

### Example 8

### Washed Sperm Kinetix^{™} Assay Amplitude

Kinetix^{™} assay AMPLITUDE *for washed cells* is related to pregnancy achievement. We have determined for washed sperm cells that, among compliant samples (i.e., samples considered to be capable of producing pregnancy based on assay results), the amplitude of Kinetix^{™} positivity in pregnancy-achieving washed sperm samples was about 5.9% - 6.7%. This can be contrasted to 3.1% positive amplitude for compliant non-pregnancy producing washed sperm.

This is an important observation for preferred IUI processing because Kinetix^{™} amplitude can vary greatly between bull ejaculates assayed as neat semen samples (bull sperm is never washed), with no effect on pregnancy. But washed human sperm, given the elapsed time, are older and it appears that those that have stopped cycling don't produce pregnancy.

Washed Sperm retrospective trial, peak amplitude vs Kinetix compliance and pregnancy outcome.

For washed replicates, the time point of the peak having the highest FcR maximum was determined, and the other replicate value for the same time point was averaged with it. Ejaculates were assigned to three groups: compliant, produced pregnancy and compliant non-pregnant and non-compliant. The FcR average maximum was determined for ejaculates which were then averaged by group to determine whether peak amplitude correlated with state, which it did as shown.

Since the same female patient received ejaculates #10 and #13, one compliant and one non-compliant, it was not possible to state which produced pregnancy. But one may suspect it was the compliant ejaculate, since no non-compliant ejaculate produced pregnancy where they could be unambiguously scored due to single insemination of the female patient with only one ejaculate. As it was not possible to know this with certainty, both the compliant and non-compliant ejaculate were included in the "pregnant" category for peak amplitude calculations. This enabled the amplitude of the pregnant group to be conservative, if the non-compliant ejaculate of the pair had been removed, the pregnant category peak amplitude would have increased, making it even more distinct from the other categories. For other calculations presented here, this patient and the ejaculates were removed.

| Patient ID | Maximum peak amplitude, 1^{st} replicate | Maximum peak amplitude, 2nd replicate | Kinetix status | Patient Outcome | Average of maximum peak amplitude, both replicates |
|---|---|---|---|---|---|
| 1 | 2 | 8.9 | Compliant | pregnant | 5.5 |
| 2 | 7 | 8.6 | Compliant | | 7.8 |
| 3 | CULLED | | Assay after IUI | | |
| 4 | 4.2 | 2.6 | Non-compliant | | 3.4 |
| 5 | 1.6 | 1.7 | Compliant | | 1./ |
| 6 | 3.78 | 2.8 | Compliant | | 3.3 |
| 7 | 4.36 | 2.4 | Non-compliant | | 3.4 |
| 8 | 9.8 | 12.2 | Non-compliant | | 11 |
| 9 | 6.2 | 2.4 | Compliant | | 4.3 |
| 10 | 4.12 | 3.0 | Non-compliant | Same woman as #13, pregnant | 3.6 |
| 11 | 2.4 | 2.2 | Compliant | | 2.3 |
| 12 | 3.7 | 1.8 | Compliant | | 2.8 |

| 13 | 14.5 | 11.1 | Compliant | Same woman as #10, pregnant | 12.8 |
|---|---|---|---|---|---|
| 14 | 6.5 | Not done | Non-compliant | | 6.5 |
| 15 | 1.8 | 1.8 | Compliant | | 1.7 |
| 16 | 1.2 | 0.9 | Compliant | | 1.1 |
| 17 | 1.4 | Not done | Non-compliant | | 1.4 |
| 18 | 1.8 | Not done | Compliant | pregnant | 1.8 |
| 19 | 1 | Not done | Non-compliant | | 1.0 |

Average peak amplitude for different FcR-state inseminations and pregnancy outcomes

| FcR State at IUI and Pregnancy Outcome | # Samples | Average Maximum Peak Amplitude |
|---|---|---|
| Compliant Pregnant | 4 | 5.9% |
| Compliant Not Pregnant | 8 | 3.1% |
| Non-compliant | 6 | 4.5% |

### References

Agarwal, A., Mulgund, A., Hamada, A., and Chyatte, M.R. (2015) A unique view on male infertility around the globe. Reprod. Biol. And Endocrin. 13:37-45.

Ahlgren, M., Bostrom, K., Malmqvist, R. (1975), "Sperm transport and survival in women with special reference to the Fallopian tube", The Biology of Spermatozoa (eds. Hafez, E.S.E., and Thibault, C.G.), pp 63-73, Karger Medical and Scientific Publishers.

Alukal, J.P. and Lipshultz, L.I. (2008), "Safety of assisted reproduction, assessed by risk of abnormalities in children born after use of in vitro fertilization techniques", Nature Clin. Practice 5(3): 140-150.

Cohen-Dayag, A., Tur-Kaspa, I., Dor, J., Mashiach, S. and Eisenback, M. (1995) Sperm capacitation in humans is transient and correlates with chemotactic responsiveness to follicular factors. Proc. Natl. Acad. Sci. 92: 11039-11043.

Fischer, B., and Bavister, B.D. (1993), "Oxygen tension in the oviduct and uterus of rhesus monkeys, hamsters and rabbits", J. Reprod. and Fert. 99: 673-679.

Guthrie, H.D., and Welch, G.R. (2012), "Effects of reactive oxygen species on sperm function", Theriogenology 78(8): 1700-1708.

Kobayashi, H., Hiura, H., John, R., Sato, A., Otsu, E., Kobayashi, N., Suzuki, R., Suzuke, F., Hyashi, C., Utsonomiya, T., Yaegashi, N., and Arima, T., (2009), "DNA methylation errors at imprinted loci after assisted conception originate in the parental sperm", Eur. J. Human Genet. 17: 1582-1591.

Sedor J, Callahan HJ, Perussia B, Lattime EC, Hirsch IH. Soluble Fc γ RIII (CD16) and immunoglobulin G levels in seminal plasma of men with immunological infertility. J. Androl. 14, 187-193 (1993).

Lu, J.-C., Huang, Y.-F., Lu, N.-Q. Antisperm Immunity and Infertility. Expert Rev. Clin. Immunol. 4(1):113-126 (2008).

Pituch-Noworolska, A., et al. The Role of Shed Fc Receptor in the Regulation of Lymphocyte Response to Phytohaemagglutinin (PHA). Immunology 55:693-701 (1985).

## Claims

1. An *in vitro* method for determining an optimum time for utilizing a semen sample for insemination or storage comprising the steps of:
i. providing a semen sample from a male subject to be tested and incubating the sample under controlled conditions of a constant pH and a temperature of from about 0° C to about 37° C;
ii. selecting a marker that is indicative of fertility status of sperm, wherein expression of the marker correlates with fertility status of the sperm, and wherein the marker is Fc receptor (FcR);
iii. detecting the level of expression of the FcR by sperm in the semen sample at a plurality of time points that are at least 10 minutes apart;
iv. determining the time point at which the sperm in the semen sample express the FcR at about a maximum or a predetermined level that correlates to approaching a maximum of FcR expression of the sperm; and
v. selecting a time point where the sperm display a minimum level of expression of the Fc receptor (FcR), and thereby providing an optimum time for administering the semen sample for insemination or freezing to provide improved pregnancy results, wherein the time point of step (v) is from about 20 minutes before to about 20 minutes after the minimum level of expression of the FcR.

2. The *in vitro* method of claim 1, where the controlled condition includes incubating the semen at a temperature of about 12° C to about 37° C.

3. The *in vitro* method of claim 1, wherein at step (iii) the plurality of time points are at least 15 minutes apart.

4. The *in vitro* method of any one of claims 1 to 3, wherein the level of FcR expression is detected in sperm, wherein the sperm are comprised in at least one of semen, vaginal fluid, media, or combination thereof.

5. The *in vitro* method of any one of claims 1 to 4, wherein the FcR is a lectin protein.

6. The *in vitro* method of any one of claims 1 to 5, wherein the lectin is a fucose-binding lectin protein.

7. The *in vitro* method of claim 1, wherein the FcR is selected from the group consisting of: CD16A; CD16a antigen; CD16B; Fc fragment of IgG low affinity IIIa receptor; Fc fragment of IgG, low affinity III, receptor for (CD16); Fc fragment of IgG, low affinity IIIa, receptor (CD16a); Fc fragment of IgG, low affinity IIIb, receptor (CD16b); Fc gamma Receptor 3; Fc gamma Receptor III; Fc gamma receptor III-A; Fc gamma receptor IIIa; Fc gamma receptor IIIb; Fc-gamma receptor III-2 (CD 16); Fc-gamma receptor IIIb (CD 16); Fc-gamma receptor IIIb (CD16); fc-gamma RIII; Fc-gamma RIII-alpha; fc-gamma RIII-beta; Fc-gamma Rllla; fc-gamma Rlllb; FcgammaRIIIA; FCGR3; FCGR3A; FCGR3B; FCGRIII; FCR-10; FCRIII; FCRIIIA; igG Fc receptor III-1; igG Fc receptor III-2; immunoglobulin G Fc receptor III; neutrophil-specific antigen NA; and RP11-5K23.1.

## Patentansprüche

1. Ein *in vitro-Verfahren* zur Bestimmung eines optimalen Zeitpunkts zur Verwendung einer Samenprobe zur Besamung oder Lagerung umfassend die Schritte:
i. Bereitstellung einer Samenprobe von einem zu untersuchenden männlichen Probanden und Inkubation der Probe unter kontrollierten Bedingungen eines konstanten pH-Werts und einer Temperatur von ungefähr 0 °C bis ungefähr 37 °C;
ii. Auswahl eines Markers, der den Fertilitätsstatus von Sperma anzeigt, wobei die Expression des Markers mit dem Fertilitätsstatus von Sperma korreliert, und wobei der Marker der Fc-Rezeptor (FcR) ist;
iii. Nachweis der Menge der Expression des FcR durch Sperma in der Samenprobe zu einer Mehrzahl von Zeitpunkten, die wenigstens 10 Minuten auseinander liegen;
iv. Bestimmung des Zeitpunkts, zu dem das Sperma in der Samenprobe den FcR ungefähr maximal exprimiert oder eine vorbestimmte Menge, die mit der Annäherung der maximalen FcR Expression des Spermas korreliert; und
v. Auswahl eines Zeitpunkts, zu dem das Sperma eine minimale Menge des Fc-Receptors (FcR) exprimiert, und dadurch das Bereitstellen eines optimalen Zeitpunkts zur Verabreichung der Samenprobe zur Besamung oder zum Einfrieren zur Bereitstellung verbesserter Schwangerschaftsergebnisse,
wobei der Zeitpunkt von Schritt (v) bei ungefähr 20 Minuten vor bis ungefähr 20 Minuten nach der minimalen Menge der Expression des FcR liegt.

2. Das *in vitro*-Verfahren von Anspruch 1, wobei die kontrollierte Bedingung das Inkubieren des Samens bei einer Temperatur von ungefähr 12° C bis ungefähr 37° C umfassen.

3. Das *in vitro*-Verfahren von Anspruch 1, wobei in Schritt (iii) die Mehrzahl der Zeitpunkte mindestens 15 Minuten auseinander liegen.

4. Das *in vitro*-Verfahren von einem der Ansprüche 1 bis 3, wobei die Menge der FcR Expression in Sperma nachgewiesen wird, wobei das Sperma in wenigstes einem von Samen, Vaginalflüssigkeit, Medium oder einer Kombination davon umfasst ist.

5. Das *in vitro*-Verfahren von einem der Ansprüche 1 bis 4, wobei der FcR ein Lectin-Protein ist.

6. Das *in vitro*-Verfahren von einem der Ansprüche 1 bis 5, wobei das Lectin ein Fukose-bindendes Lectin-Protein ist.

7. Das *in vitro*-Verfahren von Anspruch 1, wobei der FcR aus der Gruppe ausgewählt ist, bestehend aus: CD16A; CD16a Antigen; CD16B; Fc-Fragment des IgG low affinity IIIa Rezeptors; Fc-Fragment des IgG low affinity III Rezeptors für (CD16); Fc-Fragment des IgG low affinity IIIa Rezeptors (CD16a); Fc-Fragment des IgG low affinity IIIb Rezeptors (CD16b); Fc gamma Rezeptor 3; Fc gamma Rezeptor III; Fc gamma-Rezeptor III-A; Fc-gamma Rezeptor IIIa; Fc-gamma Rezeptor IIIb; Fc-gamma-Rezeptor III-2 (CD 16); Fc-gamma-Rezeptor IIIb (CD 16); Fc-gamma-Rezeptor IIIb (CD16); Fc-gamma-RIII; Fc-gamma-RIII-alpha; Fc-gamma-RIII-beta; Fc-gamma-Rllla; Fc-gamma-RIIIb; Fc-gamma RIIIA; FCGR3; FCGR3A; FCGR3B; FCGRIII; FCR-10; FCRIII; FCRIIIA; IgG Fc Receptor III-1; IgG Fc Fezeptor III-2; Immunglobulin G Fc Rezeptor III; Neutrophil-spezifisches Antigen NA; und RP11-5K23.1.

## Revendications

1. Procédé *in vitro* pour déterminer un moment optimal pour utiliser un échantillon de sperme pour une insémination ou un stockage, comprenant les étapes suivantes :
i. fourniture d'un échantillon de sperme d'un sujet mâle à tester et incubation de l'échantillon dans des conditions contrôlées à un pH constant et une température d'environ 0 °C à environ 37 °C ;
ii. sélection d'un marqueur qui indique un état de fertilité des spermatozoïdes, dans lequel l'expression du marqueur est corrélé avec l'état de fertilité des spermatozoïdes, et dans lequel le marqueur est un récepteur Fc (FcR) ;
iii. détection du niveau d'expression du FcR par des spermatozoïdes dans l'échantillon de sperme à une pluralité de moments qui sont espacés d'au moins 10 minutes ;
iv. détermination du moment où les spermatozoïdes dans l'échantillon de sperme expriment le FcR à environ un maximum ou à un niveau prédéterminé qui est corrélé avec l'approche d'un maximum de l'expression du FcR du sperme ; et
v. sélection d'un moment où les spermatozoïdes présentent un niveau minimal d'expression du récepteur Fc (FcR), et établissement par ce biais d'un moment optimal pour l'administration de l'échantillon de sperme pour insémination ou congélation pour obtenir des résultats de grossesse améliorés,
dans lequel le moment de l'étape (v) se situe entre environ 20 minutes avant et environ 20 minutes après le niveau minimal d'expression du FcR.

2. Procédé *in vitro* selon la revendication 1, dans lequel la condition contrôlée inclut une incubation du sperme à une température d'environ 12 °C à environ 37 °C.

3. Procédé *in vitro* selon la revendication 1, dans lequel, à l'étape (iii), la pluralité de moments sont espacés d'au moins 15 minutes.

4. Procédé *in vitro* selon l'une quelconque des revendications 1 à 3, dans lequel le niveau d'expression de FcR est détecté dans des spermatozoïdes, dans lequel les spermatozoïdes sont compris dans au moins un parmi du sperme, un fluide vaginal, des milieux ou une combinaison de ceux-ci.

5. Procédé *in vitro* selon l'une quelconque des revendications 1 à 4, dans lequel le FcR est une protéine de lectine.

6. Procédé *in vitro* selon l'une quelconque des revendications 1 à 5, dans lequel la lectine est une protéine de lectine se liant au fucose.

7. Procédé *in vitro* selon la revendication 1, dans lequel le FcR est sélectionné parmi le groupe constitué de : CD16A, antigène CD16A, CD16B, récepteur de type IIIa de faible affinité de fragment Fc d'IgG, récepteur de type III de faible affinité de fragment Fc d'IgG (CD16), récepteur de type IIIa de faible affinité de fragment Fc d'IgG (CD16a), récepteur de type IIIb de faible affinité de fragment Fc d'IgG (CD16b), récepteur Fc gamma de type 3, récepteur Fc gamma de type III, récepteur Fc gamma de type III-A, récepteur Fc gamma de type IIIa, récepteur Fc gamma de type IIIb, récepteur Fc gamma de type III-2 (CD 16), récepteur Fc gamma de type IIIb (CD 16), récepteur Fc gamma de type IIIb (CD16), RIII fc-gamma, RIII-alpha Fc-gamma, RIII béta fc-gamma, RIIIa Fc-gamma, RIIIb fc-gamma, RIIIA Fc gamma, FCGR3, FCGR3A, FCGR3B, FCGRIII, FCR-10, FCRIII, FCRIIIA, récepteur de type III-1 de Fc d'igG, récepteur de type III-2 de Fc d'igG, récepteur de type III de Fc d'immunoglobuline G, antigène NA spécifique à un neutrophile et RP11-5K23.1.
